# EUROPÄISCHE PATENTSCHRIFT

(11) **EP 1 239 049 B1**
(45) Veröffentlichungstag und Bekanntmachung des Hinweises auf die Patenterteilung: **22.11.2006**
(21) Anmeldenummer: 02005450.8
(22) Anmeldetag: 08.03.2002
(51) Int. Cl.: C12Q 1/26, G01N 33/557, G01N 33/58

(54) **Wasserstoffperoxid-Bestimmung mittels Oxidasen und Lanthanoid-Liganden-Komplexen**
Hydrogen peroxide determination using oxidases and lanthanoide-ligand complexes
Détermination de l'eau oxygenée avec des oxidases et des complexes lanthanoide-ligand

(30) Priorität: 09.03.2001 DE 10111392
(43) Veröffentlichungstag der Anmeldung: 11.09.2002
(73) Patentinhaber: Active Motif Chromeon GmbH, 93105 Tegernheim (DE)
(72) Erfinder: Wolfbeis, Otto, 93053 Regensburg (DE); Dürkop, Axel, 93047 Regensburg (DE)
(74) Vertreter: Dey, Michael

(56) Entgegenhaltungen:
- WO-A-94/02486
- DE-C- 19 813 247
- RAKICIOGLU Y ET AL: "Increased luminescence of the tetracycline-europium(III) system following oxidation by hydrogen peroxide." JOURNAL OF PHARMACEUTICAL AND BIOMEDICAL ANALYSIS, Bd. 20, Nr. 1-2, Juni 1999 (1999-06), Seiten 397-399, XP002240025 ISSN: 0731-7085
- SAIKI ET AL: "Use of tetracycline as complexing agent in radiochemical separations" JOURNAL OF RADIOANALYTICAL CHEMISTRY, Bd. 64, Nr. 1-2, 1981, Seiten 83-116, XP009009682
- HADI ET AL: "Complexation of some antibiotics with lanthanum (III)" EGYPT. J. CHEM., Bd. 39, Nr. 1, 1996, Seiten 49-58, XP001147291

## Beschreibung

Die vorliegende Erfindung betrifft biologische Bestimmungsverfahren unter Verwendung von Enzymen aus der Gruppe der Oxidasen und unter Verwendung von optischen Indikatoren aus der Gruppe der Lanthanoiden-Liganden-Komplexe.

Enzymatische Verfahren spielen eine bedeutende Rolle in der Bioanalytik. Wichtige Fragestellungen sind beispielsweise der Nachweis der Anwesenheit eines bestimmten Enzyms oder die quantitative Bestimmung der Aktivität von Enzymen. Aber auch Substrate, die durch Enzyme umgesetzt werden, können durch enzymatische Verfahren nachgewiesen oder bestimmt werden. Häufig durchgeführte Verfahren umfassen beispielsweise den Nachweis und die Bestimmung von Substraten wie z. B. Glucose, Alkohol, Cholesterin und Triglyceriden. Schließlich können auch Hemmer bzw. Aktivatoren von Enzymen nachgewiesen oder bestimmt werden, indem man deren reaktionsverlangsamenden bzw. reaktionsbeschleunigenden Einfluss quantitativ erfasst. Eine Zusammenfassung findet sich in "*Enzymatic Methods of Analysis",* G. G. Guilbault, Pergamon Press, 1970.

Zwei Arten von Enzymen werden bei enzymatischen Verfahren bevorzugt verwendet, Dehydrogenasen und Oxidasen. Dehydrogenasen bilden beispielsweise aus NAD⁺ das NADH, welches über seine charakteristische Absorption bei 345 nm bzw. über seine Fluoreszenz bei 455 nm nachgewiesen werden kann.

Die Aktivität von Oxidasen, die Sauerstoff verbrauchen und Wasserstoffperoxid bilden, kann beispielsweise durch Messung des Verbrauches an Sauerstoff oder der Bildung von H₂O₂ nachgewiesen werden.

Ein Beispiel für ein Enzym, das in einem Bestimmungsverfahren unter Verwendung von Oxidasen eingesetzt wird, ist die Glucoseoxidase, die Glucose zu Gluconsäurelacton und der gleichen molaren Menge an H₂O₂ umsetzt. Über die Bestimmung von H₂O₂ kann somit eine Quantifizierung der Glucose (z.B. im Blut oder in Getränken) bzw. anderer Substrate von Oxidasen erfolgen. Oxidasen können beispielsweise auch als Marker in Enzymtests oder zum Nachweis eines gebildeten Antigen-Antikörper-Komplexes (der dann ebenfalls Glucoseoxidase enthält) verwendet werden (z.B. in Immuntests vom Typ eines Sandwich-Assays). Entsprechend kann eine eingetretene Hybridisierungsreaktion zwischen zwei Nukleinsäuresträngen nachgewiesen werden.

Gegenwärtig werden verschiedene enzymatische Verfahren eingesetzt, beispielsweise elektrometrische Bestimmungsverfahren für Oxidasen und deren Substrate. Vadgama et al. beschreiben einen elektrochemischen Sensor zur Bestimmung der Reaktionsprodukte von Oxidasen. Er beruht auf der Verwendung eines Permeations-selektiven Mediums und auf dem elektrochemischen Nachweis des gebildeten Wasserstoffperoxids (EP 503 943). In WO 9953301 wird ebenfalls ein elektrochemisch-enzymatisches Verfahren beschrieben, das aber auf der Verwendung von 2 Elektroden (mit unterschiedlichen Membranen) beruht. Amperometrische Glucosemessgeräte sind kommerziell verfügbar.

In letzter Zeit haben optische Verfahren wie beispielsweise absorptiometrische und reflektorische Verfahren zur Bestimmung von Oxidasen und deren Substraten besonderes Interesse erweckt, da sie einfacher, vor allem aber spezifischer sein können. Optische Verfahren beruhen meist auf einer irreversiblen chemischen Reaktion zwischen dem von Oxidasen gebildeten Wasserstoffperoxid und einem farblosen Ausgangsstoff (z. B. Dianisidin), die zu einem stark gefärbten Produkt führt, dessen Farbintensität gemessen und mit der vorhandenen Menge an Enzym bzw. Substrat in Beziehung gesetzt werden kann.

Genovesi et al. berichten über einen photometrischen Farbtest für Glucose (*Proc. SPIE-Int. Soc. Opt. Eng.* 990 (1989) 22-28). Auch in US 5,281,395 wird ein sogenannter Teststreifen beschrieben. In WO 9805424 werden Biosensoren beschrieben, die auf der Bestimmung von Enzymsubstraten über eine irreversible Farbreaktion beruhen.

Einige dieser Verfahren werden kommerziell in sogenannten Teststreifen verwendet, wobei die Auswertung reflektometrisch erfolgt. Allerdings haben die genannten optischen Verfahren den Nachteil, dass die entstehenden Farbstoffe nicht stabil sind und das Ergebnis dadurch verfälscht werden kann (*Clin. Chem.* **19** (1973) 1227-8).

Matsubara et al. beschreiben, dass Oxo[5,10,15,20-tetra(4-pyridyl)porphyrinato]titan(IV)-Komplexe mit Harnsäure in Gegenwart des Enzyms Uricase einen gelben Komplex mit einem Absorptions-Maximum bei 432 nm bilden, der zur quantitativen photometrischen (nicht aber fluorimetrischen) Bestimmung der Harnsäure geeignet ist (*J. Pharmaceutical Soc. Jap.* 114 (1994) 48-53).

Weitere optische Verfahren sind chemilumineszente Bestimmungsverfahren für Oxidasen. Unter Chemilumineszenz versteht man die mit einer chemischen Reaktion verbundene Aussendung von sichtbarem, ultraviolettem oder gegebenenfalls infrarotem Licht unterhalb der Glühtemperatur der beteiligten Substanzen. In einem chemilumineszenten Verfahren wird beispielsweise der Umstand ausgenützt, dass das von Oxidasen gebildete Wasserstoffperoxid mit Reagenzien wie z. B. Luminol (G. L. Kok, T. P. Holler, M. B. Lopez, H. A. Nachtrieb, M. Yuan, *Environm. Sci. Technol.* 1978, *12*, 1072; T. M. Freeman, W. R. Seitz, *Anal. Chem.* 1978, *50*, 1242) oder Peroxalaten (P. van Zoomen et al., *Anal. Chim. Acta* 1985, *167*, 249) unter Aussendung einer grünen bzw. blauen Chemilumineszenz reagiert (M. A. DeLuca, W. D. McElroy (eds.) *Bioluminescence & Chemiluminescence,* Academic Press, New York, 1981). Es ist des Weiteren bekannt, dass 2-wertige Europium-Komplexe Chemilumineszenz aussenden, wenn sie mit Oxidationsmitteln behandelt werden (Elbanowski et al. *Photochem. Photobiol.* **47** (1988) 463-6; Elbanowski et al. *J. Alloys Compd.* **275-277** (1998) 225-229). Der Effekt der Chemilumineszenz ist hierbei allerdings nur beobachtbar, solange das Reagens vorhanden ist. Somit benötigen Verfahren auf der Grundlage der Messung von Chemilumineszenz zwar vorteilhafterweise keine Lichtanregungsquelle, besitzen aber den Nachteil, dass die lichtemittierende Spezies (z. B. Luminol) durch eine chemische Reaktion mit der Zeit verbraucht wird.

Weiterhin werden fluoreszente Bestimmungsverfahren für Oxidasen verwendet. Der Stand der fluoreszenz-analytischen Techniken wird in *J. Biomed. Opt.* **5** (2000) 5-16 und WO 0011205 beschrieben.

Weber et al. berichten in WO 0002048 über einen optischen Sensor für die in situ Messung von Analyten unter besonderer Betonung der Analytik der Glucose. In WO 9855869 werden weitere Glucose-Analyseverfahren beschrieben. In US 6157442 wird eine mikro-faseroptische Anordnung für die fluoreszente enzymatische Bestimmung von Glucose durch Messung des Sauerstoffverbrauches beschrieben.

Chua et al. berichten über ein Verfahren zur Messung von Plasmaglukose (*Clin. Chem.* **24** (1978) 150-2) und Tolosa et al. beschreiben ein optisches Verfahren basierend auf Lumineszenz (*Sens. Actuators, B* **B45** (1997) 93-99). In *Biosensors & Bioelectron.* **15** (2000) 69-76 wird ein fluoreszentes Verfahren beschrieben, ebenfalls unter Messung des Sauerstoffverbrauches durch das Enzym.

Andere fluoreszenz-optische Verfahren beruhen beispielsweise auf der irreversiblen Oxidation von Phenolen zu fluoreszenten Dimeren in Gegenwart von Peroxidase (*Anal. Chim. Acta* 1986, *186*, 131) oder von Mangan-Salzen (*Talanta* 1986, *33*, 914). Die entstehenden Reaktionsprodukte müssen mit UV-Licht angeregt werden (was bei chemilumineszenten Verfahren nicht erforderlich ist), um dann eine hell-blaue Fluoreszenz zu emittieren. Zhou et al. beschreiben eine Reaktion mit einem Derivat des Farbstoffes Resorufin, das bei Einwirkung von Wasserstoffperoxid ein grün fluoreszierendes Produkt ergibt (*Anal. Biochem.* 253 (1997) 162-168).

Das in enzymatischen Reaktionen gebildete Wasserstoffperoxid kann auch nachgewiesen werden, indem man es unter dem Einfluss von Peroxidase mit p-Hydroxyphenylcarbonsäuren (z. B. p-Hydroxybenzoesäure oder Homovanillinsäure) zur Reaktion bringt. Dabei bildet sich ein Dimer, das unter UV-Anregung bei 315 nm stark fluoresziert (mit einem Maximum bei ca. 415 nm), (*Sensors & Actuators* **B28** (1995) 3-7). Ein Nachteil ist, dass alle biologischen Materialien unter Anregung bei 315 nm eine stark störende Eigenfluoreszenz aufweisen. Somit weisen alle Verfahren, die auf der Bildung von fluoreszenten Produkten, die im UV angeregt werden, beruhen, starke Störeffekte durch die Fluoreszenz biologischer Materialien auf.

Die Empfindlichkeit und Selektivität des Verfahrens unter Verwendung von Homovanillinsäure kann dadurch gesteigert werden, dass man das durch Wasserstoffperoxid gebildete Dimer mit Europrium(Eu)(III)-lonen zu einem besser nachweisbaren Chelat umsetzt (*Analyst* **122** (1997) 455). Die Selektivität dieses Verfahrens beruht darauf, dass man wegen der langsamen Abklingzeit der Fluoreszenz des Eu-Chelates den Messvorgang so steuern kann, dass man zuerst die schnell-abklingende Untergrundfluoreszenz der Probe abklingen läßt und erst danach die spektral deutlich abgetrennte Fluoreszenz des Chelates misst, die somit sehr spezifisch ist. In DE 198 13 247.6 wird ein Verfahren zur Bestimmung von Glucose unter Verwendung der Enzyme Meerrettich-Peroxidase und Glucose-Oxidase sowie der Reagenzien p-Hydroxyphenylpropionsäure und Tb(EDTA) beschrieben. Auch diese Fluoreszenz klingt deutlich langsamer ab als die Untergrundfluoreszenz vieler biologischer Proben.

Dieses Verfahren wurde in *Analyst* **125** (2000) 1537-1538 genauer beschrieben. Es wurden Oxidase-Substrate bestimmt, indem man zur Untersuchungslösung als erstes Reagenz die Oxidase zugab, als zweites Reagenz die p-Hydroxyphenylpropionsäure (pHPPS) und als drittes Reagens das Enzym Peroxidase. Das von der Oxidase gebildete Wasserstoffperoxid reagiert unter dem Einfluss der Peroxidase mit dem pHPPS zu einem Dimer, das mit dem 4. Reagens (einem Lanthaniden-lon) einen lumineszenten Komplex bildet, dessen Fluoreszenz durch Zugabe des 5. Reagenzes (Cäsiumchlorid) verstärkt wird. Diese Verfahren sind zwar empfindlich, aber irreversibel und aufgrund des Bedarfes von 5 Reagenzien sehr aufwendig.

Die bisher beschriebenen enzymatischen Verfahren weisen die Nachteile auf, dass sie (a) entweder photometrisch durchgeführt werden müssen, was in stark gefärbten Lösungen sehr schwierig ist, oder dass (b) die Untergrundfluoreszenz so stark ist, dass alle anderen Signale überlagert werden oder dass (c) die Messung nur indirekt (nämlich über die Messung des Verbrauchs von Sauerstoff) erfolgt. Letzteres besitzt den Nachteil, dass der Sauerstoff-Partialdruck oft nicht bekannt ist bzw. während der Messung oder von Probe zu Probe schwankt. Die der vorliegenden Erfindung zugrundeliegende Aufgabe bestand darin, ein Verfahren bereitzustellen, bei dem die Nachteile des Standes der Technik zumindest teilweise beseitigt sind.

Die der Erfindung zugrundeliegende Aufgabe wurde gelöst durch die Bereitstellung eines Verfahrens zur Bestimmung von enzymatisch gebildetem Wasserstoffperoxid, dadurch gekennzeichnet, dass man Wasserstoffperoxid, welches von einem Enzym, ausgewählt aus der Gruppe der Wasserstoffperoxid bildenden Oxidasen, gebildet wird, mit Hilfe eines Lanthanoid-Liganden-Komplexes bestimmt.

Überraschenderweise wurde gefunden, dass während der katalytischen Aktivität von Oxidasen die Lumineszenz geeigneter anwesender Lanthanoid-Liganden-Komplexe verändert werden. Insbesondere betrifft die vorliegende Erfindung ein Verfahren zur Bestimmung von enzymatisch gebildetem Wasserstoffperoxid, worin Wasserstoffperoxid, welches von einem Enzym, ausgewählt aus der Gruppe der Wasserstoffperoxid-bildenden Oxidasen, gebildet wird, mit Hilfe eines Lanthanoid-Liganden-Komplexes bestimmt wird, dadurch gekennzeichnet, dass die Änderung in der Lumineszenz des Lanthanoid-Liganden-Komplexes bestimmt wird, indem man den Lanthanoid-Liganden-Komplex mit Licht der Wellenlängen zwischen 300 und 450 nm bestrahlt und die Änderung in der Abklingzeit der Emission oder der Intensität der Emission bei Wellenlängen größer als 500 nm gemessen wird.

Somit kann die eine Aktivität von mindestens einem Enzym, ausgewählt aus der Gruppe der Wasserstoffperoxid-bildenden Oxidasen bestimmt werden, wobei man das gebildete Wasserstoffperoxid mit Hilfe eines Lanthanoid-Liganden-Komplexes bestimmt.

Die durch Oxidasen bewirkte Freisetzung von Wasserstoffperoxid bewirkt eine Änderung in den optischen, insbesondere den lumineszenz-optischen Eigenschaften von Indikatorsubstanzen wie beispielsweise Lanthanoid-Liganden-Komplexe und kann mit Hilfe des Lanthanoid-Liganden-Komplexes nachgewiesen oder quantitativ bestimmt werden. Damit sind beispielsweise Enzyme, Enzymaktivitäten, Enzymsubstrate, Enzymhemmer oder Enzymaktivatoren auf verbesserte Weise nachweisbar bzw. bestimmbar. Durch die Verwendung von Oxidasen als Marker z.B. in immunologischen oder genetischen Nachweis-Verfahren sind darüber hinaus auch Antigene und Nucleinsäure-Oligomere nachweisbar bzw. bestimmbar.

Als Oxidasen bezeichnet man Enzyme aus der Gruppe der Oxidoreductasen, die Redoxreaktionen mit molekularem Sauerstoff als Elektronenakzeptor bewirken. Wenn vier Elektronen auf Sauerstoff übertragen werden, so entsteht Wasser oder Kohlendioxid, werden zwei Elektronen transferiert, so bildet sich Wasserstoffperoxid (H₂O₂).

"Wasserstoffperoxid bildende Oxidasen" sind alle Enzyme aus der Gruppe der Oxidoreduktasen, die ihr Substrat unter Verbrauch von Sauerstoff und unter Bildung von Wasserstoffperoxid oxidieren. Beispiele für bevorzugt im erfindungsgemäßen Verfahren eingesetzte Oxidasen sind Glucoseoxidase, Galaktoseoxidase, Cholesterinoxidase, Sarkosinoxidase, Xanthinoxidase, Bilirubinoxidase, Aminoxidase, Aminosäureoxidase, Alkoholoxidase, Lactatoxidase, Pyruvatoxidase und Uricase.

Die vorliegende Erfindung offenbart ein neues Verfahren zur einfachen Bestimmung der Aktivität von Wasserstoffperoxid bildenden Oxidasen und der Konzentration von Biomolekülen durch Verwendung eines Lanthanoid-Liganden-Komplexes. Vorteilhafterweise ist im erfindungsgemäßen Verfahren im Gegensatz zu Verfahren, die im Stand der Technik beschrieben sind, neben der Oxidase nur ein zusätzliches weiteres Reagenz, nämlich der Lanthanoid-Liganden-Komplex erforderlich. Mit dem erfindungsgemäßen Verfahren kann eine qualitative oder quantative Bestimmung durchgeführt werden. Beispielsweise können Enzyme, Enzymaktivitäten, Enzymsubstrate, Enzymhemmer, Enzymaktivatoren, Antigene oder Nukleinsäure-Oligomere bestimmt werden.

Die Bestimmung kann beispielsweise in biologischem Gewebe, in mikrobiellen oder viralen Kulturen oder in Vertiefungen von Mikrotiterplatten erfolgen.

Das Enzym aus der Gruppe der Oxidasen kann im erfindungsgemäßen Verfahren in freier Form, als Oxidase-markierter Antikörper oder als Oxidase-markierte Oligonukleinsäure vorliegen.

Die vorliegende Erfindung umfasst somit qualitative optische Nachweisverfahren und quantitative optische Bestimmungsverfahren für Enzymaktivitäten, Enzymsubstrate, Enzymhemmer, Enzymaktivatoren, Antigene oder Nukleinsäure-Oligomere unter Verwendung mindestens eines Enzyms aus der Gruppe der Oxidasen, dadurch gekennzeichnet, dass man das von Oxidasen, von Oxidase-markierten Antikörpern oder von Oxidase-markierten Oligonukleinsäuren während ihrer enzymatischen Aktivität gebildete Wasserstoffperoxid mit Hilfe eines Reagenzes, bestehend aus einem dreiwertigen Lanthanoidion, bevorzugt einem lon der Elemente Europrium, Terbium, Holmium, Dysprosium, Erbium oder Samarium, und einem organischen Liganden nachweist, wobei das Reagens durch Wasserstoffperoxid eine messbare Änderung der optischen Eigenschaften erfährt, deren Größe beispielsweise in einem bekannten oder vorab ermittelten Verhältnis zur Konzentration des zu bestimmenden Enzyms, Enzymsubstrats, Enzymhemmers, Antigens oder Oligomers stehen kann.

In einer bevorzugten Ausführungsform des erfindungsgemäßen Verfahrens erfolgt die Bestimmung von Enzymaktivitäten, worin ein Substrat in mehreren Schritten durch Enzyme, wovon mindestens eines der Gruppe der Oxidasen ist, abgebaut wird und dadurch verursachte Bildung von Wasserstoffperoxid mit Hilfe eines Lanthanoid-Liganden-Komplexes nachgewiesen oder bestimmt wird und diese zur Bestimmung der Enzymaktivität des ersten Enzyms in der Enzymkaskade herangezogen wird.

Das erfindungsgemäße Verfahren kann beispielsweise zum Nachweis oder zur Bestimmung der Aktivität von Oxidasen eingesetzt werden. Oxidasen sind Enzyme, die Sauerstoff als Zweitsubstrat oxidieren und Wasserstoffperoxid produzieren. Im Enzymkatalog (E.C.) besitzen sie die Nummer 1. X.3, wobei die Zahl 1 anzeigt, dass es sich um ein Enzym aus der Gruppe der Oxidoreduktasen handelt, die Zahl X steht für die jeweilige Substratgruppe und die Zahl 3 zeigt an, dass es sich um eine Oxidase handelt. Häufig werden Oxidasen der Substratgruppen 1 bis 14 bestimmt bzw. nachgewiesen.

Eine bevorzugte Ausführungsform der Erfindung zur Bestimmung der Aktivität von Oxidasen ist dadurch gekennzeichnet, dass ein zugegebenes Substrat durch die Oxidase abgebaut wird und die dadurch verursachte Bildung von Wasserstoffperoxid mit Hilfe eines Lanthanoid-Liganden-Komplexes nachgewiesen oder bestimmt und zur Bestimmung der Aktivität der Oxidase herangezogen wird. In einer weiteren Ausführungsform kann eine Bestimmung der Aktivität eines Enzymes erfolgen, worin ein zugegebenes Substrat in mehreren Schritten durch Enzyme, von denen mindestens eines aus der Gruppe der Oxidasen ist, abgebaut wird und dass die dadurch verursachte Bildung von Wasserstoffperoxid mit Hilfe eines Lanthanoid-Liganden-Komplexes bestimmt wird und zur Bestimmung der Aktivität des ersten Enzyms in der Enzymkaskade herangezogen wird.

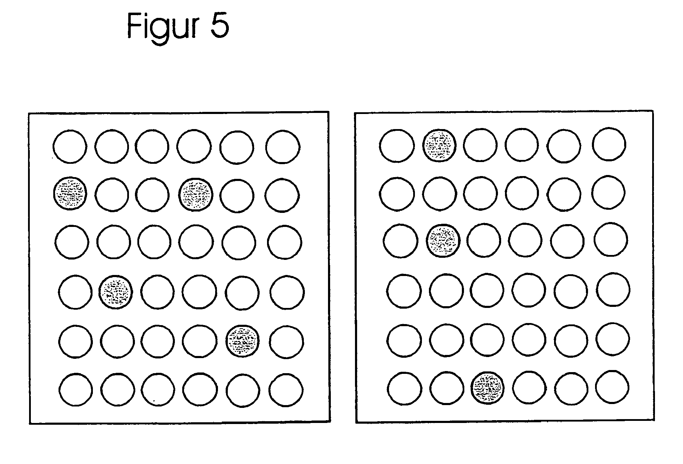

Zum erfindungsgemäßen Nachweis der Anwesenheit eines Enzyms wird zunächst ein Lanthanoid-Liganden-Komplex zugegeben, anschließend das entsprechende Enzymsubstrat. Dann wird die Fluoreszenz bei einer Wellenlänge zwischen 300 und 450 nm, vorzugsweise 330 bis 415 nm, angeregt und die Emissionsintensität bei Wellenlängen größer als 500 nm, beispielsweise von ca. 600 bis 630 nm, detektiert. In Figur 5 ist anhand der Enzyme Glucoseoxidase und Galkatoseoxidase beispielhaft der Nachweis der Enzymaktivität in Vertiefungen von Mikrotiterplatten durch Zugabe des Lanthanoid-Liganden-Komplexes und Substrat und anschließender fluormetrischer Vermessung der Mikrotiterplatte gezeigt.

Eine weiteren bevorzugte Ausführungsform des erfindungsgemäßen Verfahrens ist die Bestimmung von Enzymsubstraten.

Bevorzugt werden Substrate aus der Gruppe der Oxidasen bestimmt. Als Oxidasen bezeichnet man Enzyme aus der Gruppe der Oxidreduktanten, die Sauerstoff als zweites Substrat akzeptieren. Oxidasen oxidieren ihr Substrat unter Verbrauch von Sauerstoff und unter Bildung von Wasserstoffperoxid.

Geeignete Oxidasen für diese Ausführungsform der vorliegenden Erfindung sind beispielsweise Glucoseoxidase, Galaktoseoxidase, Cholesterinoxidase, Sarcosinoxidase, Xanthinoxidase, Aminoxidase, Aminosäureoxidase, Alkoholoxidase, Lactatoxidase, Pyruvatoxidase oder Urikase. Darüber hinaus können alle beliebigen Wasserstoffoxid bildenden Oxidasen verwendet werden. Das erfindungsgemäße Verfahren kann zur Bestimmung von Substraten, die direkt durch Oxidasen umgesetzt werden, verwendet werden. Beispielhafte Substrate sind Glucose, Lactat, Cholesterin, Bilirubin oder Alkohol. Beispielsweise wird das Substrat Glucose ("Blutzucker") durch das Enzym Glucoseoxidase nach folgender Reaktionsgleichung oxidiert:

C₆H₁₂O₆ + O₂ → C₆H₁₀O₆ + H₂O₂

(Glucose + Sauerstoff → Gluconsäurelacton + Wasserstoffperoxid).

Pro umgesetztem Molekülglucose wird ein Molekül Wasserstoffperoxid gebildet. Über die Messung der gebildeten Menge an Wasserstoffperoxid kann man somit Glucose bestimmen. Die Bestimmung kann beispielsweise kinetisch erfolgen, das bedeutet, dass man die Kinetik der Bildung des Wasserstoffperoxids über die Zeit verfolgt, bevorzugt über einen Zeitraum von 1 bis 5 Minuten.

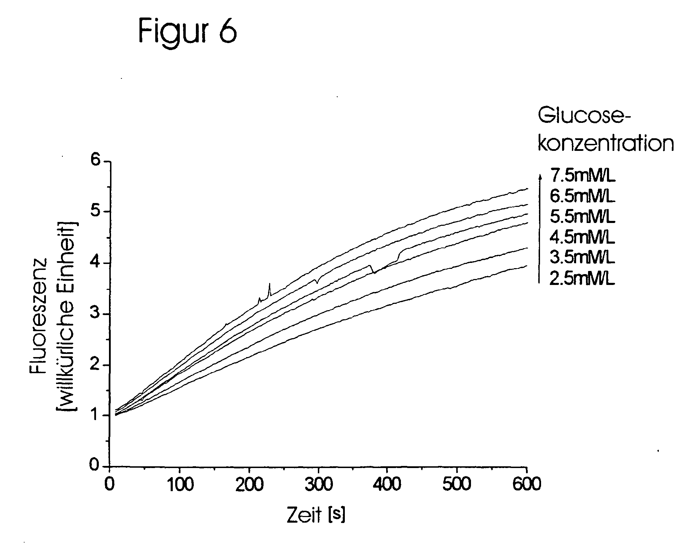

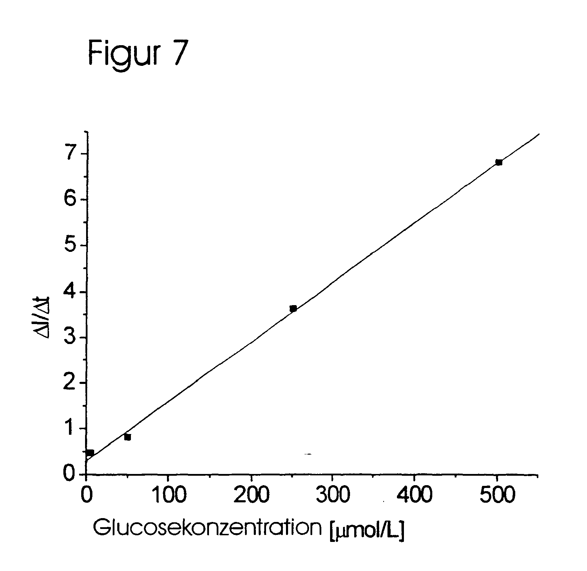

Die erfindungsgemäßen Lanthanoid-Liganden-Komplexe erweisen sich als geeignet für die Bioanalytik von Substraten von Oxidasen. In Figur 6 ist eine Änderung der Emission über die Zeit als Funktion der in einer Probe vorliegenden Glucosekonzentration gezeigt. Aus der Auftragung der Änderung der Emission pro Zeiteinheit (Δ|(Δt) gegen die Glucosekonzentration ergibt sich, dass Glucose mit dem erfindungsgemäßen Verfahren in einem Konzentrationsbereich bevorzugt zwischen 2 und 50 mMol/l quantitativ bestimmt werden kann. Dies ist in Figur 7 gezeigt.

Beispiele für die Bestimmung von Enzymsubstraten mittels des erfindungsgemäßen Verfahrens sind die Bestimmung oder der Nachweis von Glucose in Blut, Serum, Speichel, interstitieller Flüssigkeit, alkoholischen oder nicht-alkoholischen Getränken (oder deren Vorprodukten) oder in Bioreaktoren unter Verwendung von Glucoseoxidase.

Unter Verwendung von Lactatoxidase kann beispielsweise mittels des erfindungsgemäßen Verfahrens das Substrat Lactat in Blut, Serum, interstitieller Flüssigkeit oder in Bioreaktoren nachgewiesen oder bestimmt werden. Weiterhin können die Substrate Cholesterin oder Bilirubin in Blut oder Serum unter Verwendung von Cholesterinoxidase bzw. Bilirubinoxidase bestimmt werden.

Ebenso kann Alkohol in Blut, Speichel, Serum, interstitieller Flüssigkeit, alkoholischen oder nicht-alkoholischen Getränken (oder deren Vorprodukten) oder in Bioreaktoren bestimmt werden mit Hilfe des erfindungsgemäßen Verfahrens zur Bestimmung von Enzymsubstraten unter Verwendung von Alkoholoxidase.

Auch Substrate, die nicht direkt durch Oxidasen umgesetzt werden, sind einer Bestimmung mittels des erfindungsgemäßen Verfahrens unter Verwendung von Lanthanoid-Liganden-Komplexen zugänglich. Hierbei wird das Substrat zuerst durch eine oder mehrere Nicht-Oxidasen zu einem Produkt umgesetzt, welches wiederum ein Substrat für eine Oxidase ist und von dieser unter Bildung von Wasserstoffperoxid weiter umgesetzt werden kann. Ein Beispiel für eine Enzymkaskade dieser Art ist im Folgenden für die klinisch signifikante Substanz Creatinin dargestellt:

Creatinin + Creatininamidohydrolase → Creatin

Creatin + Creatinase → Sarcosin + Harnstoff

Sarcosin + **Sarcosin-Oxidase** + O₂ → Glycin + Formaldehyd + Wasserstoffperoxid

Die vorliegende Erfindung umfasst somit auch ein Verfahren zum Nachweis oder zur Bestimmung eines Enzymsubstrats, worin das zu bestimmende Substrat zuerst durch mindestens ein Enzym umgesetzt wird, das nicht der Klasse der Oxidasen angehört, und das letzte der Produkte dieser Enzymkaskade durch eine Oxidase umgesetzt wird. Ein Beispiel hierfür ist eine Ausführungsform, worin das erste Enzym der Enzymkaskade CreatininAmidohydrolase, das zweite Enzym der Enzymkaskade Creatinase und das dritte Enzym der Enzymkaskade Sarcosinoxidase ist, wobei das Verfahren zum Nachweis oder zur quantitativen Bestimmung von Creatinin in Körperflüssigkeiten eingesetzt werden kann.

Vorteilhafterweise ist das erfindungsgemäße Verfahren zur Bestimmung von Creatinin einfacher handhabbar, nachweis- und quantifizierbar, da im Gegensatz zu den im Stand der Technik beschriebenen Verfahren (Goren et al., Clinical Chemistry 32 (1986) Seiten 548 bis 551) neben dem Lanthanoid-Liganden-Komplex keine weiteren Enzyme wie beispielsweise Peroxidase oder Reagenzien, wie beispielsweise Anisidin, Aminoantipyrin oder 2,4,6-Tribrom-3-hydroxybenzoesäure zugegeben werden müssen.

Weiterhin umfasst die vorliegende Erfindung ein Verfahren zum Nachweis oder zur quantitativen Bestimmung von Enzymhemmern, worin der Reaktions-verlangsamende Einfluss eines Enzymhemmers auf den durch eine Oxidase bewirkten Abbau eines Enzymsubstrates unter Freisetzung von Wasserstoffperoxid mit Hilfe eines Lanthanoid-Liganden-Komplexes nachgewiesen oder quantitativ bestimmt wird.

Ein weiterer Gegenstand der vorliegenden Erfindung ist ein Verfahren zum Nachweis oder zur quantitativen Bestimmung von Enzymhemmern, worin der Reaktions-verlangsamende Einfluss eines Enzymhemmers auf den durch ein oder mehrere Enzyme, von denen mindestens eines aus der Gruppe der Oxidasen ist, bewirkten Abbau eines Enzymsubstrats und Freisetzung von Wasserstoffperoxid mit Hilfe eines Lanthanoid-Liganden-Komplexes nachgewiesen oder quantitativ bestimmt wird und zum Nachweis oder zur Bestimmung des Enzymhemmers des ersten Enzyms der Enzymkaskade eingesetzt wird. Bevorzugterweise ist das erste Enzym einer solchen Enzymkaskade eine Protease oder Peptidase.

Das erfindungsgemäße Verfahren zur Bestimmung von Enzymhemmern kann beispielsweise zum Screening der Wirksamkeit von potenziellen Enzym-hemmenden Substanzen durchgeführt werden. Ein solches Screening ist beispielsweise dafür geeignet, um potenzielle Pharmaka zu finden. Der Enzymhemmer kann auch eine toxische Substanz sein und das erfindungsgemäße Verfahren kann zum Nachweis oder zur quantitativen Bestimmung von toxischen Substanzen in biologischen Proben, in Nahrungsmitteln oder in Umweltproben eingesetzt werden.

Weiterin betrifft die vorliegende Erfindung ein Verfahren zum Nachweis oder zur quantitativen Bestimmung von Enzymaktivatoren, worin der reaktionsbeschleunigende Einfluss eines Enzymaktivators auch den durch eine Oxidase bewirkten Abbau eines Enzymsubstrats unter Freisetzung von Wasserstoffperoxid mit Hilfe eines Lanthanoid-Liganden-Komplexes nachgewiesen oder quantitativ bestimmt wird.

Enzymaktivatoren, die mittels des erfindungsgemäßen Verfahrens bestimmt werden können, sind beispielsweise ein- oder zweiwertige Metallionen.

In einer weiteren Ausführungsform erfolgt mittels des erfindungsgemäßen Verfahrens ein Nachweis oder eine quantitative Bestimmung von Enzymaktivatoren, worin der reaktionsbeschleunigende Einfluss eines Enzymaktivators auf den durch ein oder mehrere Enzyme, von denen mindestens eines aus der Gruppe der Oxidasen ist, bewirkten Abbau eines Enzymsubstrats oder Freisetzung von Wasserstoffperoxid mit Hilfe eines Lanthanoid-Liganden-Komplexes nachgewiesen oder quantitativ bestimmt wird und zum Nachweis oder zur Bestimmung des Enzymaktivators des ersten Enzyms der Enzymkaskade eingesetzt wird.

Eine weitere bevorzugte Ausführungsform der vorliegenden Erfindung betrifft ein Verfahren zum Nachweis oder zur quantitativen Bestimmung von Antigenen. Geeignete Oxidasen, wie beispielsweise Glucoseoxidase oder Galactoseoxidase können in optischen Immunoassays als Marker eingesetzt werden. Typischerweise wird ein Antikörper mit einer Oxidase markiert (im Folgenden gekennzeichnet mit *). Findet der so markierte Antikörper (*AK) nun ein entsprechendes Antigen (AG), so kommt es zur Bildung eines *AK-AG-Komplexes, der auch die Oxidase umfasst und der über deren Aktivität nachgewiesen werden kann (Papkovsky et al., Anal. Chem. 71 (1999) Seiten 1568-1573). In einem im Stand der Technik beschriebenen Verfahren zur Bestimmung der Aktivität der Glucoseoxidase wird ein Sauerstoffsensor eingesetzt und der durch die enzymatische Aktivität verursachte Sauerstoffverbrauch gemessen. Diese Methode ist nur dann genau, wenn der Sauerstoffgehalt von Probe und Standard gleich sind. Allerdings ist dies nicht immer gewährleistet.

Mit Hilfe der erfindungsgemäßen Lanthanoid-Liganden-Komplexe, die als Indikatoren für Wasserstoffperoxid eingesetzt werden, ist es nun erstmalig möglich, in Immunoassays vom Typ des ELISA die Bildung von Wasserstoffperoxid direkt und vorteilhafterweise auch bei variabler Sauerstoffsättigung nachzuweisen.

Die vorliegende Erfindung betrifft somit auch ein Verfahren zum Nachweis oder zur quantitativen Bestimmung von Antigenen, worin ein Oxidase-markierter Antikörper in einem immunanalytischen Verfahren eingesetzt wird und nach erfolgter ein- oder mehrmaliger Antigen-Antikörper-Bindung und Zugabe von Enzymsubstrat das durch die Oxidase gebildete Wasserstoffperoxid nachgewiesen oder bestimmt wird und zum Nachweis oder zur Bestimmung des Antigens eingesetzt wird.

Die vorliegende Erfindung umfasst auch Verfahren zum Nachweis oder zur quantitativen Bestimmung von Antigenen, worin ein Sandwichassay oder ein ELISA durchgeführt wird. Die Bestimmung oder der Nachweis von Antigenen kann beispielsweise in Blut, Serum, Speichel, interstitieller Flüssigkeit, alkoholischen oder nicht alkoholischen Getränken (oder deren Vorprodukten) in Umweltproben oder in Bioreaktoren erfolgen. Weiterhin kann ein immunhistochemischer Nachweis von Antigenen erfolgen, worin ein Oxidase-markierter Antikörper an ein in einem Gewebsschnitt befindliches Antigen bindet und der Ort der Bindung durch Zugabe eines Lanthanoid-Liganden-Komplexes und eines Enzymsubstrates sichtbar gemacht wird, bevorzugt wird er optisch mit Hilfe eines Mikroskops sichtbar gemacht.

In einer weiteren bevorzugten Ausführungsform des erfindungsgemäßen Verfahrens erfolgt ein Nachweis oder eine quantitative Bestimmung von Nukleinsäureoligomeren mittels Hybridisierungsassays. Beim Nachweis genetisch veränderter DNA kommt Hybridisierungsassays eine große Bedeutung zu. Ein Nachweis genetisch veränderter DNA kann beispielsweise in der medizinischen Diagnose, in der Forensik oder beim Nachweis gentechnisch veränderter Nahrungsmittel wünschenswert sein. Bei Hybridisierungsassays wird die eintretende (oder ausbleibende) Bildung eines Doppelstrangs aus zwei komplementären oder nicht komplementären Einzelsträngen nachgewiesen. Eine Hybridisierung ist in komplexen Proben photometrisch nicht oder nur unter großen technischem Aufwand möglich, aus diesem Grund verwendet man zum Nachweis fluoreszente Markierungsmethoden.

Durch die Markierung einer DNA oder eines Teilstrangs einer DNA mit einer Oxidase hat man, ähnlich wie beim Immunoassay, die Möglichkeit eines Nachweises einer eingetretenen Hybridisierung, indem man über die enzymatische Aktivität das gebildete Wasserstoffperoxid mittels der Lanthanoid-Liganden-Komplexe nachweist. Beispielsweise wird in einer bevorzugten Ausführungsform des erfindungsgemäßen Verfahrens ein Amino-modifiziertes Oligomer mit einer Oxidase markiert. Wenn dieses erste, markierte Oligomer (Oli-1) auf sein entsprechendes Gegenstück (Oli-2) trifft, kommt es zur Bildung eines Duplexes, der auch die Oxidase enthält und der über die Aktivität der Oxidase nachgewiesen werden kann.

Die vorliegende Erfindung umfasst auch ein Verfahren zum Nachweis oder zur quantitativen Bestimmung von Nukleinsäureoligomeren, worin ein Nukleinsäureeinzelstrang mit einer Oxidase markiert wird und deren Aktivität nach erfolgter Hybridisierung oder Umhybridisierung und nach Zugabe von Enzymsubstrat über das gebildete Wasserstoffperoxid optisch nachgewiesen wird und zum Nachweis der quantitativen Bestimmung einer Nukleinsäuresequenz eingesetzt wird. Zum Nachweis oder zur quantitativen Bestimmung von Nukleinsäureoligomeren werden bevorzugt die Oxidasen Glucoseoxidase oder Galactoseoxidase verwendet.

Eine Bestimmung von Nukleinsäureoligomeren kann beispielsweise in Blut, Sperma, Speichel, Nahrungsmitteln, Pflanzen oder Saatmaterial, Erbgut, Bakterien, Viren oder Bioreaktoren erfolgen.

Im Verfahren der vorliegenden Erfindung kann die Oxidase als freie Oxidase, als Oxidase-markierter Antikörper oder als Oxidase-markiertes Oligomer in gelöster Form in der zu bestimmenden Lösung vorlegen und/oder der Lanthanoid-Liganden-Komplex kann in gelöster Form eingesetzt werden.

Die Oxidase, der Oxidase-markierte Antikörper oder das Oxidase-markierte Oligomer und/oder der Lanthanoid-Liganden-Komplex kann auch in immobilisierter Form vorliegen, beispielsweise immobilisiert auf flächigen Elementen wie beispielsweise der Boden einer Mikrotiterplatte, auf oder in Partikeln oder auf einem Lichtwellenleiter, bevorzugt einem faseroptischen Lichtwellenleiter, wobei die immobilisierte Oxidase bevorzugt in unmittelbarem Kontakt mit der zu bestimmenden Probe ist. Eine Immobilisierung kann durch jedes bekannte System erfolgen, bevorzugt erfolgt eine Immobilisierung über das Streptavidin-Biotin oder über das Avidin-Biotinsystem.

Eine Ausführungsform des Verfahrens ist dadurch gekennzeichnet, dass sich Oxidase, Oxidase-markierter Antikörper oder Oxidase-markiertes Oligomer und der Lanthanoid-Liganden-Komplex in gelöster Form zusammen mit der zu untersuchenden Probe in einer Vertiefung einer Mikrotiterplatte befinden und die Änderung der optischen Eigenschaften des Lanthanoid-Liganden-Komplexes mittels eines Mikrotiterplattenlesers oder mittels Bild-gebender Verfahren, bevorzugt fluoreszenter Bild-gebender Verfahren bestimmt wird. In einer anderen Ausführugsform befinden sich Lanthanoid-Liganden-Komplex und/oder Oxidase, Oxidase-markierter Antikörper oder Oxidase-markiertes Oligomer in immobilisierter Form in den Vertiefungen der Mikrotiterplatte, wobei die Bestimmung der Änderung der optischen Eigenschaften mittels eines Mikroplattenlesers oder mittels Bildgebender fluoreszenter Verfahren erfolgt.

Wenn zur Immobilisierung Partikel verwendet werden, so können Oxidase, Oxidase-markierte Antikörper oder Oxidase-markiertes Oligomer und/oder Lanthanoid-Liganden-Komplex in oder auf den Partikeln immobilisiert werden. Die Partikel können durch jedes geeignete Markierungsverfahren markiert werden, bevorzugt erfolgt eine Fluoreszenzmarkierung. Die Partikel können auch einen magnetischen Kern besitzen.

Geeignete Partikel besitzenen einen Durchmesser von 0,1 bis 20 µm, mehr bevorzugt 0,1 bis 10 µm und besonders bevorzugt von 1 bis 5 µm.

Die Partikel können in heterogenen Immuntests oder Gentests oder in fließzytometrischen Nachweisverfahren eingesetzt werden.

In einer bevorzugten Ausführungsform des erfindungsgemäßen Verfahrens wird ein Fließsystem eingesetzt, das eine mechanische Zuführung von Probenmaterial, Lösungsmittel und/oder Enzymen, Enzym-markierten Antikörpern oder Enzym-markierten Oligomeren und/oder des Lanthanoid-Liganden-Komplexes vorsieht. In einer Ausführungsform des Verfahrens erfolgt die Durchführung mittels eines Fließsystems, wobei Probenmaterial und Lösungsmittel mechanisch zugeführt werden und Lanthanoid-Liganden-Komplex und/oder Enzym, Enzym-markierter Antikörper oder Enzymmarkiertes Oligomer sowohl über das Fließsystem zugeführt als auch in immobilisierter Form vorliegen können.

Das erfindungsgemäße Verfahren kann auch durchgeführt werden, wenn der Lanthanoid-Liganden-Komplex, das Enzym, oder beide nicht in Lösung, sondern immobilisiert vorliegen, beispielsweise in einem Biosensor. Lanthanoid-Liganden-Komplex und/oder die Oxidase können beispielsweise in einer Polymermembran eingebettet oder oberflächlich immobilisiert vorliegen. Geeignete Polymere halten den Lanthanoid-Liganden-Komplex bzw. das Enzym so zurück, dass sie nicht ausgewaschen werden und lassen die Eindiffusion des Enzymsubstrates zu, damit es im Inneren der Membran zu einer Bindung kommen kann.

Dünne Polymerschichten werden im Stand der Technik beispielsweise in irreversiblen Farbtests eingesetzt und als Teststreifen bezeichnet (Sonntag, Trockenchemie: Analytik mit Träger-gebundenen Reagenzien, Thieme-Verlag, Stuttgart, 1988). Solche dünnen Schichten besitzen den Vorteil, dass sie die Untersuchung optisch intransparenter Proben (z.B. Blut) ermöglichen, da die Eigenfarbe der Probe nicht mehr stört, was im Gegensatz zum Test in fluider Lösung steht, wo eine Untersuchung stark gefärbter Untersuchungsmaterialien wegen der Eigenabsorption des Probenmaterials nicht möglich ist.

In einer weiteren Ausführungsform des erfindungsgemäßen Verfahrens können die Oxidase, der Oxidase-markierte Antikörper oder das Oxidase-markierte Oligomer und/oder der Lanthanoid-Liganden-Komplex in immobilisierter Form in oder auf einer Polymermatrix vorliegen und mit der zu untersuchenden Probe in Kontakt kommen.

Die im erfindungsgemäßen Verfahren verwendete Polymermatrix ist bevorzugt für Wasserstoffperoxid durchlässig, besitzt eine Dicke von 0,05 bis 20 µm, bevorzugt von 0,1 bis 10 µm und kann beispielsweise als Biosensor in Einmaltests oder mehrmals nacheinander zur Verwendung kommen. Geeignete Polymermatrizes bestehen aus einem Hydrogel, wobei der Lanthanoid-Liganden-Komplex physikalisch oder chemisch immobilisiert in der Polymermatrix vorliegen kann.

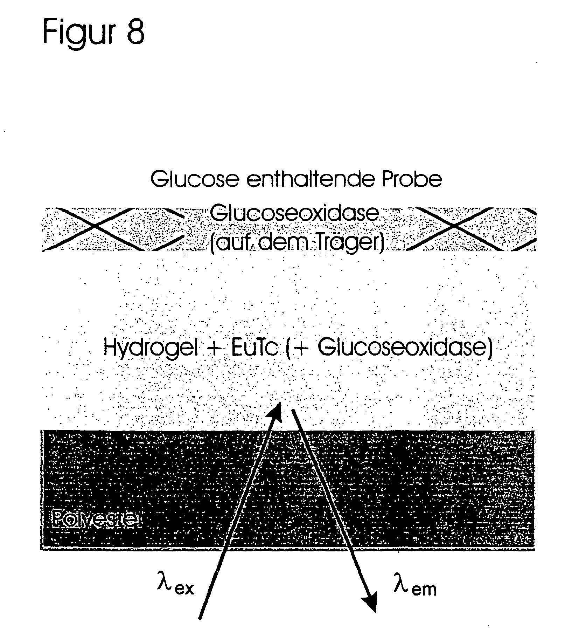

In einer Ausführungsform einer Sensormembran, befindet sich auf einer inerten, aber optisch transparenten Polyesterschicht eine einzige Sensorschicht (bestehend beispielsweise aus einem Hydrogel mit darin enthaltenem EuTc und Glucoseoxidase). In einer weiteren Ausführungsform besteht der Biosensor aus zwei Schichten, nämlich einer Schicht aus einem Hydrogel mit darin enthaltenem Lanthanoid-Liganden-Komplex und einer zweiten darüber geschichteten Schicht, bestehend aus Glucoseoxidase, die auf einem Träger (z.B. einem Nylonnetz) immobilisiert ist. Die Fluoreszenz des EuTc kann von der Polyesterseite aus abgetastet werden, indem man beispielsweise bei einer Anregungswellenlänge λex von 405 nm einstrahlt und die Emission beispielsweise bei einer Wellenlänge λem von 615 nm misst. Die Probe ("Sample") befindet sich in Kontakt mit der Sensorschicht. Wenn Glucose in die Sensorschicht eindringt, kann sich durch enzymatische Oxidation Wasserstoffperoxid bilden, dieses wird mittels eines Lanthanoid-Liganden-Komplexes nachgewiesen. In Figur 8 ist ein Querschnitt einer beispielhaften Sensormembran abgebildet.

Je nach zu bestimmenden Substrat können verschiedene Enzyme eingesetzt werden. Geeignet sind beispielsweise die Oxidasen für Lactat, Ethanol, Bilirubin, Cholesterol, verschiedene Aminosäuren (z.B. Glutamat und Lysin) sowie Amine (z.B. Catecholamine), Hydroxyphenole (z.B. Tyrosin) und (Hypo)xanthin, Harnsäuren sowie Derivate davon. Auf diese Weise können Verfahren zur Bestimmung anderer Substrate bereitgestellt werden.

Die oben beschriebenen Sensormembranen werden bevorzugt für nicht invasive Bestimmungen verwendet. Beispielsweise können Proben aus Blut, Serum, Speichel, Urin, Milch, Fruchtsäfte, Most, Fleisch, Fisch oder Bioreaktorflüssigkeiten bestimmt werden.

Darüber hinaus besteht beispielsweise im medizinischen Bereich ein Bedarf an Verfahren zur in vivo Bestimmung. Mittels des erfindungsgemäßen Verfahrens können auch in vivo Messungen vorgenommen werden. Hierzu wird eine oben beschriebene polymere Sensormembran (beispielsweise mit darin oder darauf enthaltender Glucoseoxidase) auf der Spitze eines Lichtwellenleiers, bevorzugt eines faseroptischen Lichtwellenleiters, angebracht. Auf diese Weise können faseroptische Sensoren erhalten werden, wie sie in der Literatur für andere Systeme beschrieben sind (Wolfbeis, Fiber Optic Chemical Sensors and Biosensors, CRC Press, Boca Raton, Florida, 1991).

Eine Änderung der Lumineszenz des Lanthanoid-Liganden-Komplexes kann bestimmt werden, indem man den Lanthanoid-Liganden-Komplex mit Licht der Wellenlängen zwischen 300 bis 500 nm, bevorzugt 350 bis 450 nm bestrahlt und die Änderung in der Abklingzeit der Emission oder die Änderung der Intensität der Emission bei Wellenlängen größer als 500 nm, bevorzugt bei 550 bis 650 nm, gemessen wird.

Zur Unterdrückung der Eigenfluoreszenz des Untersuchungsmaterials bzw. des Systems wird bevorzugt zuerst ein Anregungsimpuls durchgeführt und die Lumineszenz des Lanthanoid-Liganden-Komplexes nach einer Verzögerungsphase von 0,1 bis 50 µs, bevorzugt 2 bis 5 µs, bestimmt. Als Lichtquelle kann im erfindungsgemäßen Verfahren beispielsweise eine Xenonlampe, eine blaue, violette oder ultraviolette Leuchtdiode oder eine blaue, violette oder ultraviolette Laserdiode eingesetzt werden.

Die vorliegende Erfindung stellt in einer bevorzugten Ausführungsform ein Verfahren bereit, worin die Bildung von Wasserstoffperoxid kinetisch verfolgt wird und die Konzentration des Analyten durch die pro Zeiteinheit eintretende Veränderung der optischen Eigenschaften des Lanthanoid-Liganden-Komplexes nachgewiesen oder quantitativ bestimmt werden.

Eine weitere bevorzugte Ausführungsform stellt ein Verfahren bereit, worin die Bildung von Wasserstoffperoxid am Ende der Reaktion bestimmt wird und die Analyte durch die insgesamt eingetretene Änderung der optischen Eigenschaften des Lanthanoid-Liganden-Komplexes nachgewiesen oder bestimmt werden.

Während des erfindungsgemäßen Bestimmungsverfahrens wird durch Oxidase-Aktivität Wasserstoffperoxid gebildet, welches mittels eines Lanthanoid-Liganden-Komplexes nachgewiesen werden kann. Überraschenderweise erfährt das Lanthanoid-Liganden-Reagens durch Wasserstoffperoxid eine messbare Änderung der optischen Eigenschaften, insbesondere der Absorptions- und der Lumineszenzeigenschaften. Ein Vorteil der erfindungsgemäßen Lanthanoid-Liganden-Komplexe liegt in deren Stokes-Verschiebung und deren Abklingzeiten, die im Mikrosekundenbereich liegen. Damit bietet sich die Möglichkeit, über eine zeitlich auflösende Messung eine störende Untergrundfluoreszenz zuerst abklingen zu lassen und die Fluoreszenz des Lanthanoid-Liganden-Komplexes erst danach zu bestimmen, was zu extrem niedrigen Nachweisgrenzen führt.

Lanthanoid-Liganden-Komplexe weisen als Besonderheit eine Energieübetragung auf, die darin besteht, dass die vom Liganden aufgenommene photonische Energie auf das Lanthanoid-lon übertragen wird und als Lanthanoid-Emission abgegeben wird. Diese Lumineszenz ist oft schmalbandig (bzw. linienförmig), um bis zu 250 nm langwellig verschoben und hat eine Lebensdauer im Bereich von µs bis zu einigen Millisekunden. Geeignete dreiwertige lonen der Lanthanoiden-Elemente können als Marker in Lumineszenz-Immuntests verwendet werden. In einem Verfahren (DELFIA^{™}) wird der Antikörper mit einem nicht fluoreszierenden Lanthanoiden-lon markiert und nach der Bildung des Immunkomplexes mit zwei Reagenzien (Chelator und Mizellarbildner) versetzt, was zu einem deutlichen Anstieg der Fluoreszenzintensität führt. Das Verfahren wird in *Fluorescence Spectroscopy: Methods & Applications*, Wolfbeis, O. S., Ed.; Springer Verlag, Heidelberg, 1993, S. 259 - 265 beschrieben. Eine weitere Übersicht findet sich in *Anal. Chem.* 62 (1990) 1149A.

In einem anderen Immuntest wird ein Lanthanoid-Liganden-Komplex (z.B. die Phenanthrolin-dicarbonsäure BCPDA) über eine Avidin-Biotin-Bindungsreaktion zugegeben, danach wird eine Lösung von Eu(III)-Nitrat zugegeben. Nach dem Trocknen wird die Fluoreszenz ausgelesen, vorzugsweise mit Zeitverzögerungen von bis zu einigen 100 µs, um die störende Untergrundfluoreszenz des biologischen Materials zuerst abklingen zu lassen. Eine Übersicht findet sich in *Clinical Biochemistry* **21** (1988) 173.

Die in den oben beschriebenen Verfahren als Marker eingesetzte Lanthanoide müssen kovalent an ein Protein geknüpft werden. Vorteilhafterweise ist bei den erfindungsgemäßen Lanthanoid-Liganden-Komplexen keine kovalente Bindung des Lanthanoids an ein Protein bzw. einem organischen Liganden erforderlich.

Für das erfindungsgemäße Verfahren eignen sich Lanthanoid-Liganden-Komplexe mit der allgemeinen Struktur:

Ln(III)ₓ-Lig_{y},

worin
Ln(III) ein dreiwertiges Ion aus der Gruppe der Lanthanoiden ist,
x und y jeweils unabhängig ganze Zahlen von 1 bis 20 sind und das Verhältnis von x:y von 10:1 bis 1:3 beträgt, und
Lig ein an das Lanthanoidion bindender organischer Ligand ist,
wobei der Lanthanoid-Liganden-Komplex durch Wasserstoffperoxid eine Änderung seiner Absorptions- bzw. Fluoreszenzeigenschaften erfährt.

Geeignete organische Liganden besitzen die allgemeine Struktur R¹-CO-C(R²)=C(X)-R³, worin
höchstens zwei der Reste R¹, R² oder R³ H sein können,
X OH, NHR⁴, NR⁴₂ sein kann,
R¹ bis R⁴ H, ein Alkyl, ein Cycloalkyl, ein Alkanoyl, ein Cycloalkanoyl, ein Aroyl, CF₃, ein substituierter oder nicht-substituierter Alkylrest oder Alkanoylrest, OH, NH₂, Alkylamino oder Dialkylamino sein können,
wobei jeder der Reste R¹ bis R⁴ über einen substituierten oder unsubstituierten carbocyclischen oder heterocyclischen Ring mit einem der anderen Reste R¹ bis R⁴ verbunden sein kann und R¹ bis R⁴ 1 bis 30, bevorzugt 1 bis 12 C-Atome besitzt.

Bevorzugt besitzt der organische Ligand 1 bis 30 C-Atome. Geeignete Substituenten sind beispielsweise lineare oder verzweigte Alkylreste mit 1 bis 30 Kohlenstoffatomen, insbesondere mit 1 bis 8 Kohlenstoffatomen, Alkoxyreste mit 1 bis 30, bevorzugt 1 bis 8 Kohlenstoffatomen, Arylreste, bevorzugt Phenylreste, Alkylphenyl- bzw. Alkoxyphenylreste oder -SR, wobei R eine Gruppe mit 1 bis 30, insbesondere 1 bis 8 Kohlenstoffatomen ist. Weitere geeignete Substituenten sind -(CH₂-CH₂-O)ₘ bzw. -O-(CH₂-CH₂₋O)ₘ-CH₃-, wobei m eine Zahl von 1 bis 20, insbesondere von 1 bis 10 ist.

Das Lanthanoid des Lanthanoid-Liganden-Komplexes ist bevorzugt Europium, Terbium, Holmium, Dysprosium, Lanthan, Erbium oder Samarium, am meisten bevorzugt Europium, Terbium und Holmium.

Der organische Ligand des Lanthanoid-Liganden-Komplexes ist bevorzugt Benzoylaceton, Benzoyltrifluoraceton, Dibenzoylmethan, Thenoyltrifluoraceton, eine heterocyclische (ortho-Hydroxy)-Carbonsäure, ein aromatisches oder heterocyclisches ortho-Hydroxyketon oder ein Derivat davon, Hydroxychinon, eine teilweise hydrierte und substituierte Hydroxychinon-artige Verbindung, ein annelierter Carbocyclus wie beispielsweise Tetracyclin oder ein Tetracyclinderivat. Bevorzugt ist der organische Ligand nicht kovalent an das Lanthanoid gebunden.

Lanthanoid-Liganden-Komplexe können in fester, in gelöster sowie in immobilisierter Form vorliegen.

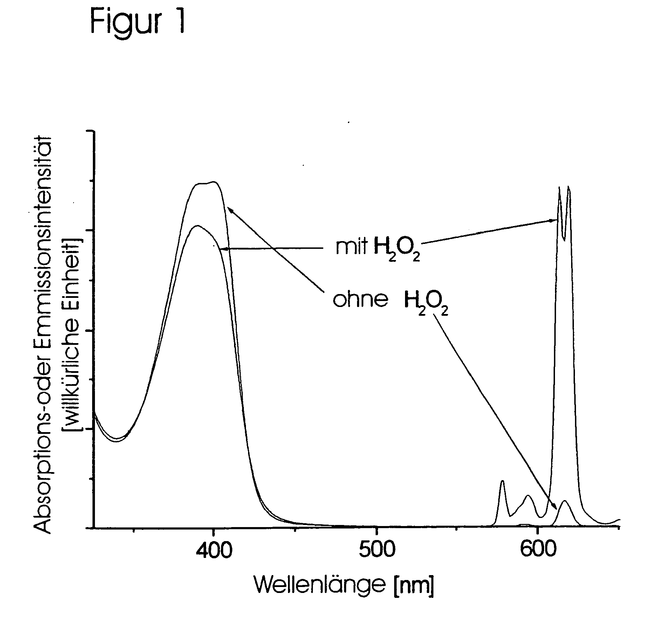

Ein besonders bevorzugter Lanthanoid-Liganden-Komplex ist ein Europium-Tetracyclin-Komplex (EuTc), wobei das stöchiometrische Verhältnis von Europoium und Tetracyclin nicht 1:1 betragen muss. Wasserstoffperoxid übt auf EuTc einen besonders starken Effekt aus. Der Effekt von Wasserstoffperoxid auf EuTc ist beispielsweise in Figur 1 dargestellt. Der EuTc zeigt die typischen spektralen Eigenschaften eines Europium-Liganden-Komplexes. Sein Absorptionsmaximum liegt bei 395 bis 405 nm, seine Absorbanz (ε) steigt in Gegenwart von Wasserstoffperoxid deutlich an, dies kann zur Bestimmung von Oxidase-katalysierten Reaktionen herangezogen werden.

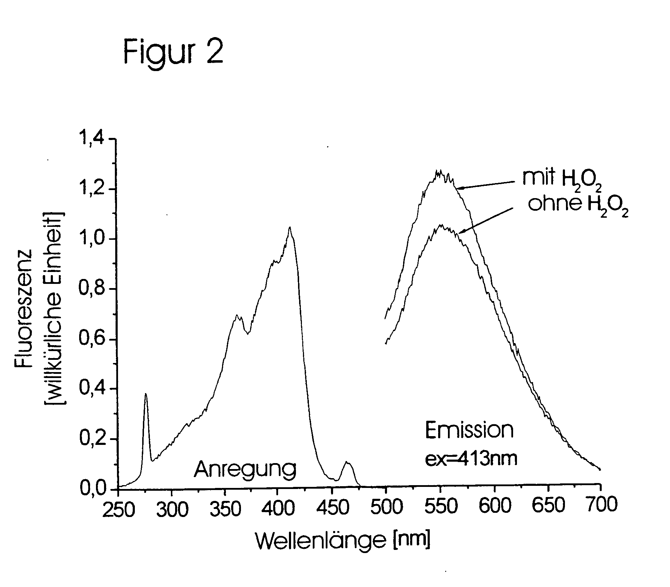

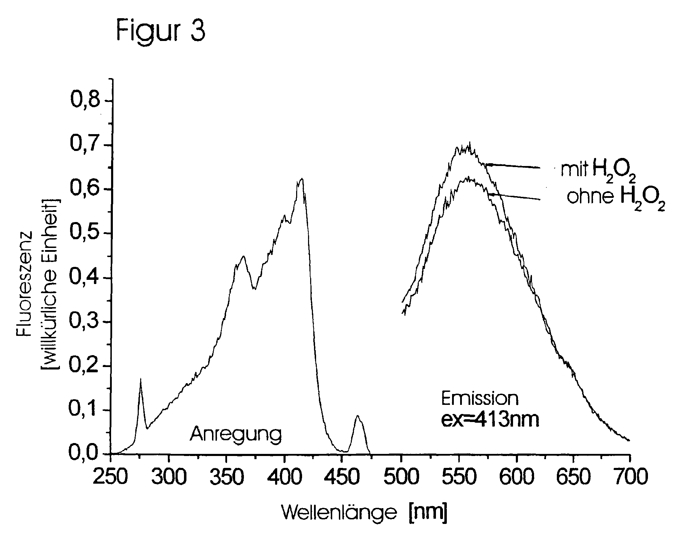

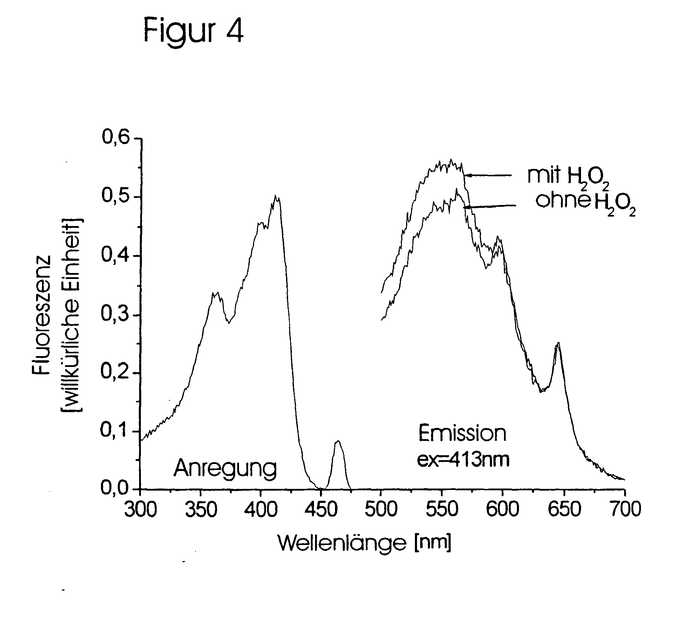

Die Emission von EuTc zeigt im sichtbaren Spektralbereich charakteristische Linien bei 615 nm. Die Lumineszenz wird bevorzugt mit Licht der Wellenlängen zwischen 300 und 450 nm angeregt, bevorzugt mittels einer Leuchtdiode. Die Lumineszenz wird durch Wasserstoffperoxid verstärkt. Auch die Fluoreszenz anderer Lanthanoid-Liganden-Komplexe, in denen das Lanthanoidion beispielsweise Dysprosium, Holmium oder Samarium ist, wird durch Wasserstoffperoxid beeinflusst, dies ist beispielsweise in den Figuren 2 bis 4 gezeigt.

Die vorliegende Erfindung wird durch die beigefügten Figuren und die folgenden Beispiele erläutert.
Fig. 1 zeigt die Absorptions und Emissionsspektren des EuTc-Komplexes in Abwesenheit und in Anwesenheit von H₂O₂. Auf Zugabe von H₂O₂ fällt zwar die Absorption bei 400 nm schwach ab, aber die Emissionsintensität bei ca. 615 nm nimmt um das bis zu 15-fache zu.
Fig. 2 zeigt den Einfluss von Wasserstoffperoxid auf das Emissionsspektrum des Dysprosium(III)-Tetracyclin-Komplexes in Wasser von pH 6.9.
Fig. 3 zeigt den Einfluss von Wasserstoffperoxid auf das Emissionsspektrum des Holmium(III)-Tetracyclinkomplexes in Wasser von pH 6.9.
Fig. 4 zeigt den Einfluss von Wasserstoffperoxid auf das Emissionsspektrum des Samarium(III)-Tetracyclinkomplexes in Wasser von pH 6.9
Fig. 5 zeigt einen Ausschnitt aus einer Mikrotiterplatte, in deren Vertiefungen ("Wells") verschiedene Enzyme (Lipasen, Dehydrogenasen, Glucoseoxidase) eingebracht worden waren. Anschliessend wurden das entsprechende Enzymsubstrat (Glucose) und ein erfindungsgemäßes Reagenz (EuTc) zugegeben. Linker Teil: Dunkle Spots stehen für helle Fluoreszenz. Diese tritt an jenen Stellen auf, an denen sich Glucoseoxidase befindet. Stellen, an denen sich Dehydrogenasen bzw. Lipasen befanden, bleiben hell (keine Fluoreszenz). Auf diese Weise erhält man Muster, die für jedes Enzymmuster (z.B. von Bakterien) typisch sind. Rechter Teil: Humanserumalbumin wurde mit verschiedenen Enzymen (beispielsweise Glucoseoxidase, markiert und in die Wells platziert. Nach Zugabe von EuTc und Glucose findet man starke Fluoreszenz nur an den Stellen, wo sich Glucoseoxidase-markiertes HSA befindet. Hingegen bleiben Wells, in denen sich HSA befindet, die mit Nicht-Oxidasen (z. B. der Glucosedehydrogenase) markiert worden war, hell.

Fig. 6 zeigt die zeitliche Zunahme der Lumineszenzintensität des Reagenzes EuTc in Gegenwart von Glucoseoxidase und einem Serum, das Glucose in verschiedenen physiologischen Konzentrationen enthält. Als analytische Information diente die Änderung der Lumineszenzintensität pro 3 min.

Fig. 7 zeigt eine Kalibrationskurve für die Bestimmung von Glucose mit Hilfe von Glucoseoxidase und dem Reagenz EuTc in Wasser bei pH 6.9 durch Auftragung der Änderung der Fluoreszenzintensität pro Zeit [ΔF/Δt] gegen die Glucosekonzentration.

Fig. 8 zeigt einen Querschnitt durch eine Sensormembran für Glucose. Auf einer inerten, aber optisch transparenten Polyester-Schicht liegt eine untere Schicht bestehend aus einem Hydrogel-Polymer und einem darin enthaltenen erfindungsgemäßen indikator (im vorliegenden Beispiel EuTc). Die darüberliegende Schicht besteht aus Glucoseoxidase, die auf einen Träger aus Nylonnetz immobilisiert worden war. Die Fluoreszenz des EuTc wird von der Polyesterseite abgetastet, indem man bei der Anregungswellenlänge λ_{exc} von 405 nm einstrahlt und die Emission bei einer Wellenlänge λₑₘ von ca. 615 nm vermisst. Die Probe ("sample") befindet sich in Kontakt mit der Sensorschicht. Wenn Glucose in die Sensorschicht eindringt, bildet sich durch enzymatische Oxidation Wasserstoffperoxid, und dieses wird durch einen Anstieg in der Fluoreszenzintensität (und in einer Abnahme der Abklingzeit) des Reagenzes angezeigt.

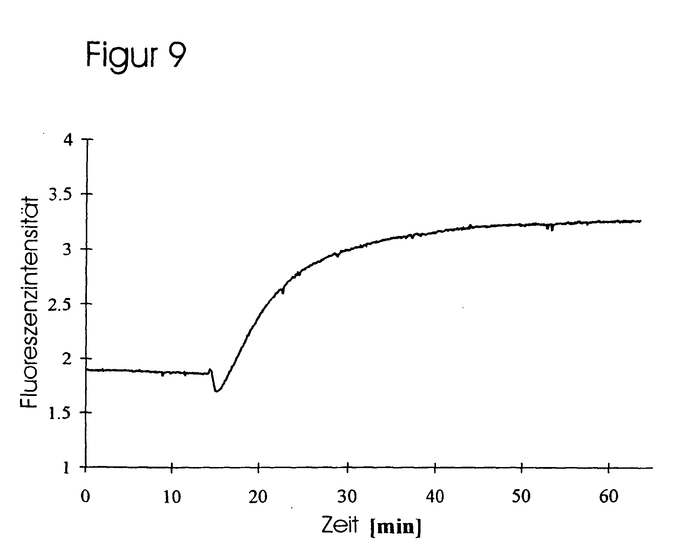

Fig. 9 zeigt das zeitliche Ansprechverhalten einer Polymer-Sensormembran (wie in Figur 8 dargestellt) mit einem darin enthaltenen Indikator (EuTc) gegenüber einer 1%-igen Lösung von Glucose in einem Puffer mit pH 6.9. Die Sensormembran war ein eine Durchflusszelle eingespannt, die sich in einem Fluorometer befand. Glucoselösung wurde durch die Zelle über die Sensormembran gepumpt und die Änderung der Fluoreszenzintensität gegen die Zeit aufgetragen. Der anfängliche Abfall resultiert aus einer eingedrungenen Luftblase.

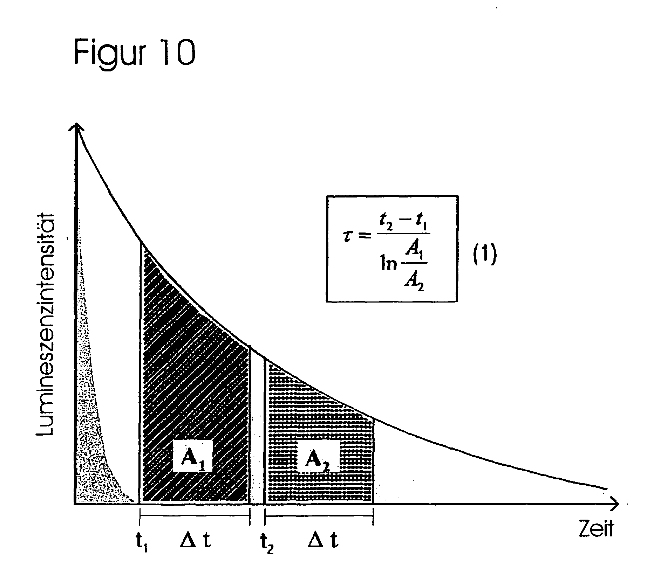

Fig. 10 zeigt eine schematische Darstellung einer zeitaufgelösten Messung zur Unterdrückung der durch biologische Materialien bedingten Untergrundfluoreszenz. Man regt mit einem kurzen Lichtpuls an, wartet danach bis zum Zeitpunkt t₁, bis zu dem die Untergrundfluoreszenz praktisch vollständig abgeklungen ist (typischerweise nach 100 - 500 ns), und öffnet danach das Messfenster. Man kann nun entweder ein integrale Fluoreszenz-Intensität A₁ bestimmen, oder aber durch Bestimmung von A₁ und A₂ (und mit Hilfe der angegebenen Gleichung) die Abklingzeit des Systems bestimmen, die ebenfalls als analytische Messgröße dienen kann. Beide Methoden dienen der Unterdrückung der störenden Untergrundfluoreszenz.

### Beispiel 1: Herstellung eines Lanthanoiden-Liganden-Komplexes (EuTc)

Zur Herstellung des Puffers löst man 1,48 g MOPS Na⁺-Salz (Fluka AG) in 490 mL destill. Wasser, stellt den pH-Wert der Lösung mit wenig 70%-iger Perchlorsäure auf pH 6,9 ein und füllt auf 500 mL Endvolumen auf. Die Reagenslösung erhält man, indem man 4,0 mg Tetracyclin-Hydrochlorid (Fluka AG) und 9,6 mg EuCl₃-Hexahydrat (Alfa) in 100 mL des obigen Puffers löst. Das Reagens kann in trockener Form erhalten werden, indem man das gelöste Reagens ohne Pufferzusatz herstellt und die Lösung danach gefriertrocknet. Andere Mengenverhältnisse zwischen Tetracyclin und Europium-lon sind möglich.

### Beispiel 2: Enzymatische Bestimmung von Glucose in Serum

*Glucose-Oxidase-Stammlösung:* Man löst 2,0 mg Glucoseoxidase (50 000 Units, Sigma) in 10 mL MOPS-Buffer.

*Bestimmungsverfahren:* In eine Fluoreszenzküvette füllt man 1 mL des Reagenzes, 1 mL des hinsichtlich Glucose zu bestimmenden Serums (mit einem Gehalt von 3 - 50 mMol/L Glucose) und danach 1 mL der Glucoseoxidase-Stammlösung. Man verfolgt die Zunahme der Fluoreszenzintensität über die Zeit ab dem Zeitpunkt der Zugabe der Glucoseoxidase. Der Anstieg der Fluoreszenz nach einer definierten Zeit, z.B. nach 3 min, ist ein Maß für die im Serum vorhandene Konzentration an Glucose. Der genaue Wert kann anhand vorab mit Hilfe von Standardlösungen ermittelter Kalibrationskurven (wie in Figur 7 gezeigt), berechnet werden.

### Beispiel 3: Bestimmung von Xanthin

*Prinzip:*

Xanthin (+ Xanthin-Oxidase) = > Harnsäure + H₂O₂

Die Menge an gebildetem Wasserstoffperoxid ist von der Konzentration an Xanthin abhängig und kann mit Hilfe eines erfindungsgemäßen Reagenzes nachgewiesen werden.

*Bestimmungsverfahren:* Man pipettiert in eine 5-mL-Küvette 1 mL der in Beispiel 1 beschriebenen Reagenz-Lösung und 1 mL einer Lösung von 4,5 Enzym-Einheiten Xanthin-Oxidase (Sigma; E.C. Nr. 1.1.3.22.) in 10 mL eines 0.015 molaren Phosphat-Puffers. Danach fügt man 1 mL der auf Xanthin zu analysierenden Lösung (die ca. zwischen 0.5 und 5 mM an Xanthin enthalten soll) und misst unmittelbar danach den Anstieg der Fluoreszenz zwischen 600 und 640 nm unter Lichtanregung bei 400-410 nm. Der Anstieg der Fluoreszenz über die Zeit ist ein Maß für die Xanthin-Konzentration. Die Daten für die Aufstellung einer Kalibrationskurve werden erhalten, indem man anstelle der zu analysierenden Lösung verdünnte Lösungen von Xanthin im gleichen Puffer (mit einem Xanthingehalt zwischen 0.1 und 10 mM) einsetzt. Eine Kalibrationskurve erhält man durch Auftragung der Fluoreszenzänderung (ΔF) pro Zeit (2-5 min) gegen die Konzentration an Xanthin.

### Beispiel 4: Bestimmung von Glucose in Serum mit Hilfe eines automatisierten Fließsystems unter Verwendung von immobilisierter Glucoseoxidase und eines Wasserstoffperoxid-Sensors

*Prinzip:*

Glucose (+ Glucoseoxidase) = = = = = > Gluconolacton + H₂O₂

*Bestimmungsverfahren*. Es wurde ein Fließinjektionssystem der Fa. Eppendorf (Hamburg) eingesetzt. Als Fließlösung diente ein HEPES-Puffer von pH 6.9. Die Probenvorbereitung erfolgte dadurch, dass die zu untersuchenden Humanseren mit HEPES-Puffer (pH 6.9) 1:1 verdünnt wurden. Die Glucoseoxidase wurde auf folgende Weise auf Agarose immobilisiert: Glucoseoxidase wurde mit dem Reagens Biotin-amidocaproat-NHS-ester (Sigma, Prod. Nr. B 2643) nach Vorschrift umgesetzt. Die so biotinylierte Glucoseoxidase (auch kommerziell erhältlich; Sigma G 7779) wurde an Agarosekörnchen ("beads"), die an der Oberfläche Avidingruppen tragen (Sigma; Produkt Nr. A 9207) immobilisiert. Die Bindung zwischen Avidin und Biotin ist sehr stark. Die resultierenden Körnchen wurden in ein kleines Plastikröhrchen gefüllt, dieses an beiden Enden mit Watte verschlossen, die so erhaltene Reaktionskammer danach an das Fließsystem angeschlossen und mit Puffer befüllt.

Die Herstellung des Wasserstoffperoxid-Sensors erfolgte dadurch, dass man eine 5%ige Lösung des Hydrogels HN80 (von Kingston Technology Inc., Dayton, NJ, USA) in Dimethylsulfoxid (DMSO) bereitete. Diese viskose Flüssigkeit wurde in einer Dicke von 100 µm auf eine Polyesterfolie aufgestrichen und danach 4 h an feuchter Luft stehen gelassen. Die entstandene klare Membran wurde mit Wasser gewaschen und war etwa 6-8 µm dick. Die Membran wurde danach in 100 mL einer Lösung gegeben, die 0.1% Europiumchlorid und 0.1% Tetracyclin enthielt. Nach 24 h wurde sie herausgenommen, gespült und war danach einsatzbereit. Der so erhaltene Film wurde an der Wand einer kleinen Durchflusszelle befestigt, und diese danach an das Fließsystem angeschlossen.

Die eigentliche Bestimmung von Glucose erfolgte dadurch, dass 100 µL der mit Puffer verdünnten Serumproben in das Fließsystem injiziert wurden. Die injizierte Proben durchliefen die Reaktionskammer (mit immobilisierter Glucoseoxidase) und unmittelbar danach die Durchflusszelle mit dem darin enthaltenen Wasserstoffperoxid-Sensor.

Das System wurde mit einer 5 mM Lösung von Glucose in einem HEPES-Puffer, der 2% Albumin enthielt, geeicht. Danach wurden jeweils 9 Proben analysiert, wobei nach jeder Probe wieder Puffer durch den Analysator gepumpt wurde, bis das Fluoreszenzsignal des Sensors wieder zum Ausgangswert zurückgekehrt war. Nach jeweils 9 untersuchten Proben wurde wieder eine Kalibration mit einem 5 mM Glucose-Standard vorgenommen.

Als analytisches Signal diente die von der in der Durchflusskammer enthaltenen Sensormembran emittierte Fluoreszenz. Die Anregung erfolgte bei 410 nm, die Fluoreszenzintensität wurde bei > 600 nm gemessen und über eine Zeit von 60 sec integriert, was der Zeit entspricht, die die Probe in der Durchflusskammer verweilt.

### Beispiel 5: Bestimmung von Lactat in Serum in einer Mikrotiterplatte

Diese Bestimmung erfolgte durch Zugabe des Enzyms Lactat-Oxidase (Sigma L-0638; Konzentration 5 mg/200 µL) zu 100 µL einer mit Puffer pH 6.9 verdünnten Serumprobe in einer Mikrotiterplatte und Nachweis des gebildeten Wasserstoffperoxids über die Fluoreszenz einer zugefügten Reagenslösung (50 µL), die in Beispiel 1 beschrieben ist.

### Beispiel 6: Multi-Enzym-Assay für Creatinin

Creatinin im Serum ist bei renaler Malfunktion deutlich erhöht.

*Prinzip:*

Creatinin (+ Creatininase) = = = > Creatin

Creatin (+ Creatinase) = = = > Sarcosin + Harnstoff

Sarcosin (+ Sarcosin-Oxidase) ===> Glycin + Formaldehyd + H₂O₂

Das gebildete Wasserstoffperoxidwird mit Hilfe eines erfindungsgemäßen Lanthanoid-Liganden-Reagenzes nachgewiesen. Ebenfalls anwesendes Creatin kann in einem zweiten Ansatz bestimmt werden, in welchem die erste der 3 Reaktionen nicht durchgeführt wird.

*Probenvorbereitung*: Die Serumprobe wird mit Puffer von pH 7.3 im Verhältnis 1:1 verdünnt.

### Bes timmungs verfahren

*(a) Creatinin* + *Creatin:* Man legt 200 µL einer Probe (mit einem Gehalt von 5 - 10 mM Creatinin) vor. Dazu gibt man zuerst 0.5 mL des Wasserstoffperoxid-Reagenzes und danach eine getrennt bereitete Lösung von 1 mg Creatininase (aus *Pseudomonas*; frei von Creatinase; mit einer Gesamtaktivität von 150 - 200 Einheiten), 10 mg Creatinase (aus *Flavobacterium;* Gesamtaktivität 100 - 200 Einheiten) und 10 mg Sarconsinoxidase (aus *Arthrobacter,* Gesamtaktivität 50 - 150 Einheiten) in 0.5 mL Phosphatpuffer, der 2% HSA enthält. Man verfolgt die Lumineszenz des Reagenzes ab der Zugabe der Enzymlösung. Die Änderung der Lumineszenz über die Zeit (3 min) ist ein Maß für die Creatininkonzentration.
*(b) Nur Creatin:* Um eventuell vorhandenes Creatin zu bestimmen, führt man die gleiche Reaktion durch, gibt aber dem System keine Creatininase zu. Dadurch wird nur das Creatin erfasst.
*(c) Nur Creatinin:* Der Wert ergibt sich aus der Differenz der beiden vorherigen Bestimmungen.

### Beispiel 7. Bestimmung von Total-Cholesterol (Multi-Enzym-Assay):

*Prinzip:*

Cholesterolester (+ Cholesterolesterase) = = = = > Cholesterol + Fettsäuren

Cholesterol (+ Cholesteroloxidase) = = = = > Cholest-4-en-3-on + H₂O₂

Die Menge an gebildetem Wasserstoffperoxid ist proportional zu der im Serum vorhandenen Konzentration an Gesamt-Cholesterol und wird mit Hilfe eines erfindungsgemäßen Reagenzes bestimmt.

### Beispiel 8: Lactat-Bestimmung (Multi-Enzym-Assay)

Prinzip:

Lactat + NAD (+ Lactatdehydrogenase) = = = > Pyruvat + NADH

Pyruvat + Phosphat (+ Pyruvatoxidase) = = = > Acetylphosphat + H₂O₂

Das gebildete Wasserstoffperoxid wird mit Hilfe eines erfindungsgemäßen Reagenzes bestimmt.

### Beispiel 9: Bestimmung von Triglyceriden (Multi-Enzym-Assay)

Eine Bestimmung von Triglyceriden wird beispielsweise zur Diagnose von Hyperlipoproteinaemien durchgeführt. Hyperlipoproteinaemien gelten als Risikofaktor für Herz- und Kreislauferkrankungen

*Prinzip:* Triglyceride werden zuerst mit Lipase verseift und das freiwerdende Glycerin danach in einer 2-Enzym-Reaktion kinetisch bestimmt.

Triglyceride (+ Lipase) = = = > Glycerin + Fettsäuren

Glycerin + ATP (+ Glycerolkinase) = = = > Glycerol-1-phosphat + ADP

Glycerol-1-phosphat (+ Oxidase) = = = > Dihydroxyaceton-Phosphat + H₂O₂

Das gebildete Wasserstoffperoxid wird mit Hilfe eines erfindungsgemäßen Reagenzes bestimmt.

### Beispiel 10: Bestimmung der Aktivität des Enzyms Alanin-Aminotransferase (ALT)

Dieses Enzym hat diagnostische Bedeutung, da es beim Auftreten verschiedener Lebererkrankungen (insbes. Hepatitis) in erhöhter Aktivität auftritt. Die Bestimmung erfolgt in einem Multi-Enzym-Verfahren über folgende Reaktionssequenz:

L-Alanin + 2-Ketoglutarat (+ ALT) = = = > Pyruvat + Glutamat

Pyruvat + Phosphat (+ Pyruvat-Oxidase) = = = > Acetylphosphat + H₂O₂

Die gebildete Menge an Wasserstoffperoxid ist der Aktivität des Enzyms ALT proportional und wird mit Hilfe eines erfindungsgemäßen Reagenzes bestimmt.

### Beispiel 11: Bestimmung der Aktivität der alkalischen Phosphatase (ALP):

Die Aktivität dieses Enzyms ist bei diversen Erkrankungen (beispielsweise Tumoren) der Leber und der Knochen stark erhöht.

*Prinzip:*

Tyrosin-Phosphat (+ ALP) = = = > Tyrosin

Tyrosin (+ Tyrosin-Decarboxylase) = = = > Tyramin

Tyramin (+ Tyramin - Oxidase) = = = > p-Hydroxyphenylacetaldehyd + H₂O₂

Die gebildete Menge an Wasserstoffperoxid ist der Aktiviät des Enzyms ALP proportional und wird mit Hilfe eines erfindungsgemäßen Reagenzes bestimmt.

### Beispiel 12: Bestimmung der Aktivität der Cholinesterase

Die Aktivität dieses Enzyms ist bei Vergiftungen mit Nervengiften deutlich erniedrigt.

*Prinzip:*

Acetylcholin (+ Cholinesterase) = = = > Acetat + Cholin

Cholin (+ Cholinoxidase) = = = > Betain + H₂O₂

Das gebildete Wasserstoffperoxid wird mit Hilfe eines erfindungsgemäßen Reagenzes bestimmt.

### Beispiel 13: Bestimmung der Aktivität der Lactatdehydrogenase

Die Aktivität dieses Enzyms ist nach einem myocardialem Infarkt stark erhöht.

*Prinzip einer selbstverstärkenden Reaktionssequenz:*

Pyruvat + NADH (+ Lactatdehydrogenase) = = = > Lactat

Lactat (+ Lactatoxidase) = = = > Pyruvat + H₂O₂

Die Menge an gebildetem Wasserstoffperoxid ist von der Aktivität der Lactatdehydrogenase abhängig und kann mit Hilfe eines erfindungsgemäßen Reagenzes bestimmt werden.

### Beispiel 14: Bestimmung von Hemmern der HIV-Proteinase

Hemmer dieses Enzyms stellen potenzielle HIV-Pharmaka dar.

*Prinzip:* Proteasen können aus Peptiden eine endständige Aminosäure (AS) abspalten. Hemmer verlangsamen die Aktivität der Protease deutlich, was sich in einer verminderten Bildung von Wasserstoffperoxid äußert.

Peptid (+ HIV-Proteinase) = = = > Aminsäure (z. B. Ala; Leu) + Rest-Peptid

Aminosäure (+ AS-Oxidase) = = => = = = > oxidierte Aminosäure (z. B. Pyruvat; α-Ketoisocaproat) + H₂O₂

Die Menge an gebildetem Wasserstoffperoxid ist von der Aktivität der HIV-Proteinase abhängig und kann mit Hilfe eines erfindungsgemäßen Reagenzes bestimmt werden. Die erste Reaktion wird durch Inhibitoren der HIV-Protease, z. B. durch Acetyl-Pepstatin, deutlich gehemmt (Richards et al., *FEBS Letters* **253** (1989) 214.)

### Beispiel 15: Bestimmung eines Antigens (HSA) über einen Immunoassay auf magnetischen Partikeln

*(a) Biotinylierung von anti-HSA:* Polyclonales anti-HSA (Ziege; Sigma Prod. Nr. A-1151) wurde nach 10-facher Verdünnung mit Phosphat-Puffer mit dem Biotinylierungs-Reagens Biotin-amidocapronsäure-sulfo-NHS-ester (Sigma, B-1022) nach der Vorschrift von Psantano et al. (*Analytical Chemistry* **65** (1993) 623) umgesetzt. Das so biotinylierte anti-HSA wurde danach an die Magnetpartikel gebunden (siehe Beispiel 15 (c)).
*(b) Markierung von anti-HSA mit Glucoseoxidase:* Die Markierung erfolgte über die Biotin-Streptavidin-Bindungsreaktion. Polyclonales anti-HSA (Ziege; Sigma Prod. Nr. A-1151) wurde nach 10-facher Verdünnung mit Phosphat-Puffer zuerst mit Streptavidin-Maleinimid (Sigma, Prod. Nr. S-9415) nach der Vorschrift von Duncan et al. in *Analytical Biochemistry* **132** (1983) 68 markiert. Das so entstandene Streptavidin/anti-HSA-Konjugat wurde nach elektrophoretischer Aufreinigung mit dem Glucoseoxidase-Markierungs-Reagens Biotinamino-Glucoseoxidase (Sigma Nr. G-7779) versetzt und elektrophoretisch gereinigt.
*(c) Immobilisierung von anti-HSA auf Magnetbeads:* Die Immobilisierung erfolgte über die Biotin-Streptavidin-Bindungsreaktion. 1 ml einer Suspension von paramagnetischen Eisenoxidpartikeln mit oberflächlich gebundenem Streptavidin (d = 1 µm; Sigma; Prod. Nr. S 2415; enthaltende etwa 1 mg immobilisiertes Streptavidin) wurden mit 1 ml einer Lösung von biotinyliertem anti-HSA (s. unter (a)) versetzt und 1 h bei Raumtemperatur stehen gelassen. Die Partikel wurden dann mit Hilfe eines Magnetseparators (Sigma, Prod. Nr. M-1292) abgetrennt.
*(d) Bestimmungsverfahren*. Die nach (c) erhaltenen Magnetpartikel wurden in 2 ml Phosphatpuffer von pH 7.0 suspendiert und mit Lösungen versetzt, die zwischen 2 und 20 µg/ml HSA enthielten, was der im Urin oder bei Microalbuminurie vorkommenden HSA-Konzentration entspricht. Nach 10 min wurden die Partikel mittels Magnetseparator abgetrennt, in 2 ml PBS resuspendiert und mit einer Lösung von 1 mg/ml des Glucoseoxidase-markierten polyclonalen anti-HSA (siehe b) versetzt. In einem derartigen Sandwich-Assay bindet das anti-HSA an das bereits am Partikel befindliche HSA. Nach nochmaliger magnetischer Trennung und Resuspension wurde eine 0.1%ige Lösung von Glucose in PBS und 1 ml einer Lösung zugegeben, die 0.1% Tetracyclin (Sigma, Prod. Nr. T-3258) und 0.1% Europiumchlorid (Fluka) enthielt. Die pro Zeiteinheit gebildete Menge an Wasserstoffperoxid kann durch den Anstieg in der Fluoreszenz bei 610- 620 nm (unter Anregung bei 405 nm) bestimmt werden und ist proportional der in der Probe enthaltenen Konzentration an HSA.

### Beispiel 16: Nachweis einer spezifischen Oligomeren-Sequenz über einen Hybridisierungs-Assay

*Prinzip:* Die Bindung eines Glucoseoxidase-markierten 16-mers an ein komplementäres 16-mer, das auf ein Agarose-Partikel immobilisiert worden war, wurde über die mittels der erfindungsgemäßen Reagenzien nachweisbare Aktivität der Glucoseoxidase nachgewiesen.

### (a) Herstellung einer Streptavidin-markierten Galactose-Oxidase

Galactose-Oxidase (E. C. 1.1.3.9) wurde in bekannter Weise mit dem Reagens Streptavidin-Maleinimid (Sigma S-9415) markiert und aufgereinigt.

### (b) Herstellung eines mit Galactose-Oxidase markierten Oligomers:

Die Immobilisierung erfolgte durch Konjugation eines biotinylierten 16-mers der Sequenz [Biotin-(CH₂)₆-ATTACCGGACTCTATA-3'] (Metabion GmbH, München) an in Beispiel 15 beschriebene Streptavidin/Glucoseoxidase-Konjugatundnachfolgendeelektrophoretische Aufreinigung.

*(c) Herstellung eines partikelgebundenen Oligomers:* Die amino-modifizierte Sequenz [3'-TAATGGCCTGAGATAT-(CH₂)₆-NH₂] wurde mit Hilfe des Kopplungsreagenzes EDAC (Sigma, E-1769) kovalent an die Carboxygruppe von in TRIS-Puffer (pH 7.5) suspendierten carboxy-modifizierten Latex-Partikeln (Bangs Labs. Inc.; d = 20 µm) konjugiert. Die Partikel wurde abzentrifugiert, gewaschen und in TRIS-Puffer resuspendiert.

### (d) Hybridisierung und Nachweis der Aktivität der Glucoseoxidase:

Die in Beispiel 15 beschriebene Partikelsuspension (1 ml) wurde mit 100 µl einer Lösung von Glucoseoxidase-markiertem Oligomer versetzt und bei 55 °C hybridisiert. Die Partikel wurden nach 1 h abzentrifugiert, gewaschen und in Puffer resuspendiert. Nach Zugabe von 500 µl einer 0.1%igen Lösung von Galactose und 500 µl einer Lösung, die 0.1% Europiumnitrat und 0.1% Tetracyclin enthielt, wurde ein deutlicher Anstieg in der Fluoreszenz bei 610-620 nm (unter Anregung bei 405 nm) festgestellt.

Dies ist Beweis für eine Hybridisierung des Glucoseoxidase-markierten Stranges. Bei Verwendung anderer Glucoseoxidase-markierter Sequenzen trat wegen der nicht eintretenden Hybridisierung (und damit der Abwesenheit von Glucoseoxidase in den Partikel) keine Bildung von Wasserstoffperoxid auf.

### Beispiel 17: Beispiel für ein zeitaufgelöste Messverfahren

Die Fluoreszenz der erfindungsgemäßen Lanthaniden-Reagenzien klingt vergleichsweise langsam ab (wie in **Fig. 10** dargestellt). Bei der Messung wird der Lanthanoiden-Komplex mit einem kurzen (0.01 - 0.1 µs) Puls einer UV-Leuchtdiode (Nichia; λ ₘₐₓ bei 375 nm) anregt. Die Integration der emittierten Lichtintensität erfolgt dann nach einer Verzögerungszeit t₁, die sich an der Abklingzeit des Lanthanoiden-Komplexes orientiert, vorzugsweise aber 10 µs nicht überschreitet. Man kann auch Abklingzeiten dadurch bestimmen, dass man das Verhältnis von 2 Flächen (A₁ und A₂) bestimmt. (*Appl. Spectrosc.* **54** (2000) 548-559). Das entsprechende Abtastschema ist in Fig. 10 dargestellt.

Die analytische Information beim erfindungsgemäßen Verfahren besteht nicht nur in der Messung der Änderung der Lumineszenzintensität, sondern auch in der Messung der Änderung der Lumineszenz-Abklingzeit. Das EuTc besitzt in Abwesenheit von Wasserstoffperoxid eine Haupt-Abklingzeit von 12,4 µs, nach Sättigung mit Wasserstoffperoxid aber eine Abklingzeit von 9,0 µs. Vorzugweise regt man bei dieser Messmethode die Fluoreszenz mit einer violetten Leuchtdiode oder einer UV-LED (Nichia) an, und bestimmt die Abklingfunktion des emittierten Lichtes, das auf der Emissionsseite mit Hilfe eines OG 570 Langpass-Filters von violetten und blauen Lichtanteilen befreit worden war.

### Beispiel 18: Herstellung eines Biosensormembran mit immobilisiertem Enzym und immobilisiertem Lanthanoid-Liganden-Indikator

Man löst 2 g eines Hydrogels (Hypan; von Hymedix, Dayton, Ohio) in 20 g Dimethylsulfoxid (Merck) und zieht die Lösung auf eine 120 µm dicke transparente Polyesterfolie (*Mylar*^{*™*}; von Goodfellow). Nach Verdunsten des Lösungsmittel über Wasser ist die verbliebene Schicht ca. 12 µm dick. Aus der beschichteten Folie wurden Spots (∅ 3-10 mm) ausgestanzt und für 5 h in eine EuTc-Reagenzlösung (siehe Beispiel 1) gelegt. Die Schicht färbte sich gelb. Die gelben Spots wurden 24 h in einem 5 mM MOPS-Puffer (ohne EuTc) liegen gelassen und waren danach gebrauchsfertig. Diese Sensormembran wird danach mit einer 2 µm dicken Schicht bedeckt, die aus 9 Gewichtsteilen p-HEMA (Poly-Hydroxyethylmethacrylate, Sigma P 3183) oder Polyacrylamid, und aus einem Gewichtsteil des Glucoseoxidase-PAA-Konjugates (Sigma, Produkt-Nr. G9255) besteht. Das Ansprechen einer derartigen Sensormembran auf eine Lösung von Glucose ist in Abb. 8 dargestellt.

### Beispiel 19: Bestimmungsverfahren für einen Enzymhemmer (Schwermetall-lon) unter Verwendung einer Biosensormembran am Boden der Vertiefung einer Mikrotiterplatte

*(a) Vorbehandlung der Mikrotiterplatte (MTP):* Eine Standard-MTP mit 96 Vertiefungen ("wells") wird am Boden mit Sensorspots beklebt, die durch Ausstanzen einer Biosensor-Fläche erhalten werden, wie sie in Beispiel 18 beschrieben ist, ausgenommen, dass die Nylon-Oberfläche nicht mit Glucose-Oxidase, sondern mit Glutamat-Oxidase (E.C. 1.4.3.11; Sigma Nr. G-0400) belegt wurde.
*(b) Reaktionsfolge:*

   Harnstoff (+ Urease) => Ammoniak + Bicarbonat

   Ammoniak + 2-Oxoglutarat + NADPH (+ Glutamat-Dehydrogenase) = > = > Glutamat + NADP

   Glutamat (+ Glutamat-Oxidase) = Abbauprodukt + H₂O₂
*(c) Bestimmung:* Man befüllt, vorzugsweise mit einem Pipettierautomaten, die mit Sensorspots versehenen Vertiefungen der MTP nacheinander mit folgenden Lösungen:
   (*) 100 µl einer 10%igen Lösung von Harnstoff in Tris-Puffer von pH 8.0;
   (*) 2 mg Glutamat-Dehydrogenase (E. C. 1.4.1.3) gelöst in 10 µl Tris-Puffer;
   (*) 1 mg NADPH (Na-Salz; Sigma N-1630) gelöst in 10 µl Puffer;
(d) 100 µl einer Umweltprobe mit einem Schwermetallgehalt (Ag, Pb, Cu, Cd, im Bereich von 0.1 - 10 mg/l;
(e) 10 mg einer Lösung von Urease (E.C. 3.5.1.5; Sigma Prod. Nr. U-1500) in 10 µl Puffer.

Man misst unmittelbar nach der Zugabe der Urease die Verlangsamung der Bildung von Wasserstoffperoxid als Folge der Hemmung durch Schwermetalle. Die maximale Bildungsgeschwindigkeit für Wasserstoffperoxid wird ermittelt, indem man eine Lösung vermisst, in der die 0.5 ml der Umweltprobe durch destilliertes Wasser ersetzt werden.

Die Menge an gebildetem Wasserstoffperoxid ist vom Gehalt der Probelösung an Schwermetallionen abhängig, da die Aktivität der Urease schon durch Spuren von Schwermetallen gehemmt wird (*Preininger et al., Biosensors & Bioelectronics* **11** (1996) 981-990). Die zeitliche Zunahme der Konzentration an Wasserstoffperoxid wird über die Lumineszenz der in den Wells befindlichen Sensorspots mit Hilfe eines von unten abtastenden Mikrotiterplattenlesers (Ascent Fluoroskan) bestimmt.

## Patentansprüche

1. Verfahren zur Bestimmung von enzymatisch gebildetem Wasserstoffperoxid, worin Wasserstoffperoxid, welches von einem Enzym, ausgewählt aus der Gruppe der Wasserstoffperoxid-bildenden Oxidasen, gebildet wird, mit Hilfe eines Lanthanoid-Liganden-Komplexes bestimmt wird,
**dadurch gekennzeichnet,**
**dass** die Änderung in der Lumineszenz des Lanthanoid-Liganden-Komplexes bestimmt wird, indem man den Lanthanoid-Liganden-Komplex mit Licht der Wellenlängen zwischen 300 und 450 nm bestrahlt und die Änderung in der Abklingzeit der Emission oder der Intensität der Emission bei Wellenlängen größer als 500 nm gemessen wird.

2. Verfahren nach Anspruch 1, worin eine Enzymkaskade aus zwei oder mehreren Enzymen, wovon mindestens eines aus der Gruppe aus Oxidasen ist, vorliegt.

3. Verfahren nach einem der vorhergehenden Ansprüche, worin mindestens eines der Enzyme aus der Gruppe der Oxidasen als freies Enzym, als Oxidase-markierter Antikörper oder als Oxidase-markierte Oligonukleinsäure vorliegt.

4. Verfahren nach einem der vorhergehenden Ansprüche, worin eine qualitative oder quantitative Bestimmung von Enzymen, Enzymaktivitäten, Enzymsubstraten, Enzymhemmern, Enzymaktivatoren, Antigenen oder Nukleinsäure-Oligomeren erfolgt.

5. Verfahren nach einem der vorhergehenden Ansprüche, worin eine Bestimmung von Glukose, Alkohol, Lactat, Bilirubin, Cholesterin, Creatinin, toxischen Substanzen, enzymhemmenden Substanzen oder ein- oder zweiwertigen Metallionen erfolgt.

6. Verfahren nach einem der vorhergehenden Ansprüche, worin eine Bestimmung von Proben aus Blut, Sperma, Speichel, interstitieller Flüssigkeit oder anderen Körperflüssigkeiten, alkoholischen oder nicht-alkoholischen Getränken oder deren Vorprodukten, Bioreaktoren, Nahrungsmitteln, Umweltproben, Pflanzen- oder Saatmaterial, Erbgut, Bakterien, Viren oder von anderen biologischen Proben erfolgt.

7. Verfahren nach einem der vorhergehenden Ansprüche, worin die Oxidase ausgewählt ist aus Glucoseoxidase, Galactoseoxidase, Bilirubinoxidase, Cholesterinoxidase, Sarcosinoxidase, Xanthinoxidase, Aminoxidase, Aminosäureoxidase, Alkoholoxidase, Lactatoxidase, Pyruvatoxidase, Uricase oder einer anderen Oxidase, die ihr Substrat unter Bildung von Wasserstoffperoxid enzymatisch umsetzt.

8. Verfahren nach einem der vorhergehenden Ansprüche zur Bestimmung von Enzymhemmern, worin der Reaktions-verlangsamende Einfluss eines Enzymhemmers auf den durch eine Oxidase bewirkten Abbau eines Enzymsubstrates unter Freisetzung von Wasserstoffperoxid mit Hilfe eines Lanthanoid-Liganden-Komplexes bestimmt wird.

9. Verfahren nach einem der vorhergehenden Ansprüche zur Bestimmung von Enzymaktivatoren, worin der Reaktionsbeschleunigende Einfluss eines Enzymaktivators auf den durch eine Oxidase bewirkten Abbau eines Enzymsubstrats unter Freisetzung von Wasserstoffperoxid mit Hilfe eines Lanthanoid-Liganden-Komplexes bestimmt wird.

10. Verfahren nach einem der vorhergehenden Ansprüche zur Bestimmung von Antigenen, worin ein Oxidase-markierter Antikörper in einem immunanalytischen Verfahren eingesetzt wird und nach erfolgter ein- oder mehrmaliger Antigen-Antikörperbindung und Zugabe von Enzymsubstrat das durch die Oxidase gebildete Wasserstoffperoxid bestimmt wird und zur Bestimmung des Antigens eingesetzt wird.

11. Verfahren nach Anspruch 10 zum immunhistochemischen Nachweis von Antigenen, worin ein Oxidase-markierter Antikörper an ein in einem Gewebsschnitt befindliches Antigen bindet und der Ort der Bindung durch Zugabe eines Lanthanoid-Liganden-Komplexes und eines Enzymsubstrates optisch sichtbar gemacht wird.

12. Verfahren nach einem der vorhergehenden Ansprüche zur Bestimmung von Nukleinsäure-Oligomeren, worin ein NukleinsäureEinzelstrang mit einer Oxidase markiert wird und deren Aktivität nach erfolgter Hybridisierung und nach Zugabe von Enzymsubstrat über das gebildete Wasserstoffperoxid optisch bestimmt wird und zur Bestimmung einer Nukleinsäuresequenz eingesetzt wird.

13. Verfahren nach einem der vorhergehenden Ansprüche, worin die Oxidase, der Oxidase-markierte Antikörper oder das Oxidase-markierte Oligomer und/oder der Lanthanoid-Liganden-Komplex in immobilisierter Form auf oder in einem Partikel, auf einem flächigen Element, auf einem Lichtwellenleiter oder auf oder in einer Polymermatrix vorliegt.

14. Verfahren nach Anspruch 13, worin die Polymermatrix aus einem für Wasserstoffperoxid durchlässigen Hydrogel besteht und in einer 0,1 bis 10 µm dicken Schicht vorliegt und als Biosensor in Einmaltests oder mehrmals nacheinander zur Verwendung kommt.

15. Verfahren nach einem der vorhergehenden Ansprüche, worin Oxidase, Oxidase-markierter Antikörper oder Oxidase-markiertes Oligomer und/oder der Lanthanoid-Liganden-Komplex in einer Mikrotiterplatte in gelöster oder immobilisierter Form vorliegen und die Änderungen der optischen Eigenschaften des Lanthanoid-Liganden-Komplexes mit Hilfe eines Mikrotiterplattenlesers oder mit Hilfe bildgebender fluoreszenter Verfahren nachgewiesen oder quantitativ bestimmt werden.

16. Verfahren nach einem der vorhergehenden Ansprüche, worin ein Fließsystem eingesetzt wird, das eine mechanische Zuführung von Probenmaterial, Lösungsmittel und/oder Enzymen oder Enzym-markierten Antikörpern oder Enzym-markierten Oligomeren und/oder des Lanthanoiden-Liganden-Komplexes vorsieht.

17. Verfahren nach einem der vorherigen Ansprüche, worin zur Unterdrückung der Eigenfluoreszenz des Untersuchungsmaterials bzw. des Systems zuerst ein Anregungspuls durchgeführt wird und die Lumineszenz des Lanthanoid-Liganden-Komplexes nach einer Verzögerungsphase von 0,1 bis 50 µs bestimmt wird.

18. Verfahren nach einem der vorhergehenden Ansprüche, worin die Bildung von Wasserstoffperoxid kinetisch verfolgt wird und die Konzentration des Analyten durch die pro Zeiteinheit eintretende Veränderung der optischen Eigenschaften des Lanthanoid-Liganden-Komplexes bestimmt wird.

19. Verfahren nach einem der vorhergehenen Ansprüche, worin die Bildung von Wasserstoffperoxid am Ende der Reaktion bestimmt wird und der Analyt durch die insgesamt eingetretene Änderung der optischen Eigenschaften des Lanthanoid-Liganden-Komplexes bestimmt wird.

## Claims

1. Method for determining enzymatically generated hydrogen peroxide wherein hydrogen peroxide that is formed by an enzyme selected from the group of hydrogen peroxide-generating oxidases is determined with the aid of a lanthanoid-ligand complex,
**characterized in that**
the change in the luminescence of the lanthanoid-ligand complex is determined by irradiating the lanthanoid-ligand complex with light at wavelengths between 300 and 450 nm and measuring the change in the decay time of the emission or the intensity of the emission at wavelength of more than 500 nm.

2. Method as claimed in claim 1, wherein an enzyme cascade comprising two or more enzymes of which at least one is from the group of oxidases is present.

3. Method as claimed in one of the previous claims, wherein at least one of the enzymes from the group of oxidases is present as a free enzyme, as an oxidase-labelled antibody or as an oxidase-labelled oligonucleic acid.

4. Method as claimed in one of the previous claims, wherein enzymes, enzyme activities, enzyme substrates, enzyme inhibitors, enzyme activators, antigens or nucleic acid oligomers are qualitatively or quantitatively determined.

5. Method as claimed in one of the previous claims, wherein glucose, alcohol, lactate, bilirubin, cholesterol, creatinine, toxic substances, enzyme-inhibiting substances or monovalent or divalent metal ions are determined.

6. Method as claimed in one of the previous claims, wherein samples of blood, sperm, saliva, interstitial fluid or other body fluids, alcoholic or non-alcoholic drinks or precursors thereof, bioreactors, foods, environmental samples, plant or seed material, hereditary material, bacteria, viruses or other biological samples are determined.

7. Method as claimed in one of the previous claims, wherein the oxidase is selected from glucose oxidase, galactose oxidase, bilirubin oxidase, cholesterol oxidase, sarcosine oxidase, xanthine oxidase, amine oxidase, amino acid oxidase, alcohol oxidase, lactate oxidase, pyruvate oxidase, uricase or another oxidase, which enzymatically convert their substrates with formation of hydrogen peroxide.

8. Method as claimed in one of the previous claims for determining enzyme inhibitors, wherein the reaction-retarding effect of an enzyme inhibitor on the degradation of an enzyme substrate caused by an oxidase with release of hydrogen peroxide is determined with the aid of a lanthanoid-ligand complex.

9. Method as claimed in one of the previous claims for determining enzyme activators, wherein the reaction-accelerating effect of an enzyme activator on the degradation of an enzyme substrate caused by an oxidase with release of hydrogen peroxide is determined with the aid of a lanthanoid-ligand complex.

10. Method as claimed in one of the previous claims for determining antigens, wherein an oxidase-labelled antibody is used in an immunoanalytical method and, after single or multiple antigen-antibody binding and addition of the enzyme substrate, the hydrogen peroxide that is formed by the oxidase is determined and used to determine the antigen.

11. Method as claimed in claim 10 for the immunohistochemical detection of antigens, wherein an oxidase-labelled antibody binds to an antigen present in a tissue section and the binding site is optically visualized by adding a lanthanoid-ligand complex and an enzyme substrate.

12. Method as claimed in one of the previous claims for the determination of nucleic acid oligomers, wherein a nucleic acid single strand is labelled with an oxidase and, after hybridization and after addition of enzyme substrate, its activity is detected optically by means of the generated hydrogen peroxide and is used to determine a nucleic acid sequence.

13. Method as claimed in one of the previous claims, wherein the oxidase , the oxidase-labelled antibody or the oxidase-labelled oligomer and/or the lanthanoid-ligand complex is present in an immobilized form on or in a particle, on a planar element, on a light waveguide or on or in a polymer matrix.

14. Method as claimed in claim 13, wherein the polymer matrix consists of a hydrogel permeable to hydrogen peroxide and is present in a 0.1 to 10 µm thick layer and is used as a biosensor in single-use tests or is used several times in succession.

15. Method as claimed in one of the previous claims, wherein oxidase, oxidase-labelled antibody or oxidase-labelled oligomer and/or the lanthanoid-ligand complex are present in a microtitre plate in a dissolved or immobilized form and the changes in the optical properties of the lanthanoid-ligand complex are detected or quantitatively determined with the aid of a microtitre plate reader or with the aid of fluorescent imaging methods.

16. Method as claimed in one of the previous claims, wherein a flow system is used in which the sample material, solvent and/or enzymes or enzyme-labelled antibodies or enzyme-labelled oligomers and/or the lanthanoid-ligand complex are transported mechanically.

17. Method as claimed in one of the previous claims, wherein the self-fluorescence of the test material or of the system is suppressed by firstly carrying out an excitation impulse and then determining the luminescence of the lanthanoid-ligand complex after a delay phase of 0.1 to 50 µs.

18. Method as claimed in one of the previous claims, wherein the formation of hydrogen peroxide is monitored kinetically and the concentration of the analyte is determined by means of the change in the optical properties of the lanthanoid-ligand complex that occur per time unit.

19. Method as claimed in one of the previous claims, wherein the formation of hydrogen peroxide is determined at the end of the reaction and the analyte is determined by the total change in the optical properties of the lanthanoid-ligand complex that occur.

## Revendications

1. Procédé pour le dosage de peroxyde d'hydrogène, formé par voie enzymatique, dans lequel le peroxyde d'hydrogène qui est formé par une enzyme, choisie dans le groupe des oxydases formant le peroxyde d'hydrogène, est dosé à l'aide d'un complexe lanthanoïde-ligand,
**caractérisé en ce que** l'on détermine la modification de luminescence du complexe lanthanoïde-ligand, en irradiant le complexe lanthanoïde-ligand avec de la lumière de longueurs d'ondes entre 300 et 450 nm, et en mesurant la modification du temps de décroissance de l'émission ou de l'intensité de l'émission à des longueurs d'ondes supérieures à 500 nm.

2. Procédé selon la revendication 1, dans lequel est présente une cascade enzymatique comportant deux enzymes ou plus, dont au moins l'une fait partie du groupe des oxydases.

3. Procédé selon l'une des revendications précédentes, dans lequel au moins l'une des enzymes du groupe des oxydases est présente sous forme d'enzyme libre, d'anticorps marqué par une oxydase ou d'acide oligonucléique marqué par une oxydase.

4. Procédé selon l'une des revendications précédentes, dans lequel on effectue un dosage qualitatif ou quantitatif d'enzymes, d'activités enzymatiques, de substrats d'enzymes, d'inhibiteurs d'enzymes, d'activateurs d'enzymes, d'antigènes ou d'acides nucléiques oligomères.

5. Procédé selon l'une des revendications précédentes, dans lequel on effectue un dosage de glucose, d'alcool, de lactate, de bilirubine, de cholestérol, de créatinine, de substances toxiques, de substances inhibant les enzymes ou d'ions métalliques mono- ou bivalents.

6. Procédé selon l'une des revendications précédentes, dans lequel on procède à un dosage sur des échantillons de sang, de sperme, de salive, de liquides interstitiels ou autres liquides corporels, de boissons alcoolisées ou non alcoolisées, ou de leurs précurseurs, de bioréacteurs, de produits alimentaires, sur des échantillons prélevés dans l'environnement, des matériaux végétaux ou des semences, sur du matériel génétique, des bactéries, des virus ou autres échantillons biologiques.

7. Procédé selon l'une des revendications précédentes, dans lequel l'oxydase est choisie parmi le glucose oxydase, le galactose oxydase, bilirubin oxydase, cholestérol oxydase, sarcosine oxydase, xanthine oxydase, amine oxydase, l'aminoacide oxydase, l'alcool oxydase, lactate oxydase, pyruvate oxydase, l'uricase ou d'autres oxydases qui transforme enzymatiquement leur substrat en formant du peroxyde d'hydrogène.

8. Procédé selon l'une des revendications précédentes pour le dosage d'inhibiteurs enzymatiques, dans lequel on détermine à l'aide d'un complexe lanthanoïde-ligand l'influence de ralentissement de la réaction d'un inhibiteur enzymatique sur la dégradation par une oxydase d'un substrat enzymatique sous libération de peroxyde d'hydrogène provoquée.

9. Procédé selon l'une des revendications précédentes pour le dosage d'activateurs enzymatiques, dans lequel on détermine à l'aide d'un complexe lanthanoïde-ligand l'influence accélératrice de réaction d'un activateur enzymatique sur la dégradation par une oxydase d'un substrat enzymatique sous libération de peroxyde d'hydrogène provoquée.

10. Procédé selon l'une des revendications précédentes pour le dosage d'antigènes, dans lequel on utilise un anticorps marqué par oxydase dans un procédé immunoanalytique, et après une ou plusieurs formations de complexe antigène-anticorps et addition de substrat enzymatique, on dose le peroxyde d'hydrogène formé par l'oxydase et on l'utilise pour le dosage de l'antigène.

11. Procédé selon la revendication 10 pour la détection immunohistochimique d'antigènes, dans lequel un anticorps marqué par oxydase se lie à un antigène se trouvant dans une coupe tissulaire et l'on visualise optiquement l'emplacement de la liaison par addition d'un complexe lanthanoïde-ligand et d'un substrat enzymatique.

12. Procédé selon l'une des revendications précédentes pour le dosage d'oligomères d'acides nucléiques, dans lequel un acide nucléique simple brin est marqué avec une oxydase et l'activité de celle-ci est déterminée optiquement, après hybridation et addition du substrat enzymatique, via l'oxyde d'hydrogène formé et utilisé pour le dosage d'une séquence d'acide nucléique.

13. Procédé selon l'une des revendications précédentes, dans lequel l'oxydase, l'anticorps.marqué par oxydase ou l'oligomère marqué par oxydase et/ou le complexe lanthanoïde-ligand se présentent sous forme immobilisée sur ou dans une particule, sur un élément plat, sur un guide d'onde de lumière ou sur ou dans une matrice polymère.

14. Procédé selon la revendication 13, dans lequel la matrice polymère est un hydrogel perméable au peroxyde d'hydrogène et se présente sous la forme d'une couche d'une épaisseur de 0,1 à 10 µm et est utilisée comme biocapteur dans des tests uniques ou dans des tests multiples consécutifs.

15. Procédé selon l'une des revendications précédentes, dans lequel l'oxydase, l'anticorps marqué par oxydase ou l'oligomère marqué par oxydase et/ou le complexe lanthanoïde-ligand se présentent sous forme dissoute ou immobilisée dans une plaque de microtitrage et les modifications des propriétés optiques du complexe lanthanoïde-ligand sont déterminées quantitativement à l'aide d'un lecteur de plaque de microtitrage ou à l'aide de procédés d'imagerie de fluorescence.

16. Procédé selon l'une des revendications précédentes, dans lequel on utilise un système d'écoulement qui prévoit une amenée mécanique de l'échantillon, du solvant et/ou d'enzymes ou d'anticorps marqués par enzyme ou d'oligomères marqués par enzyme et/ou du complexe lanthanoïde-ligand.

17. Procédé selon l'une des revendications précédentes, dans lequel, afin de supprimer la fluorescence propre du matériau ou du système à analyser, on effectue d'abord une pulsion d'excitation et on détermine la luminescence du complexe lanthanoïde-ligand après une phase de temporisation de 0,1 à 50 µs.

18. Procédé selon l'une des revendications précédentes, dans lequel la formation du peroxyde d'hydrogène est suivie cinétiquement et la concentration de l'analyte est déterminée par la modification des propriétés optiques du complexe lanthanoïde-ligand par unité de temps.

19. Procédé selon l'une des revendications précédentes, dans lequel on détermine la formation de peroxyde d'hydrogène à la fin de la réaction, et l'analyte est dosé par la modification globale des propriétés optiques du complexe lanthanoïde-ligand.
